# EUROPEAN PATENT APPLICATION

(11) **EP 1 489 180 A1**
(43) Date of publication of application: **22.12.2004**
(21) Application number: 03743606.0
(22) Date of filing: 05.03.2003
(51) Int. Cl.: C12N 15/12, A61K 45/00, C07K 14/47, C12N 5/10, C12N 1/15, C12N 1/19, C12N 1/21, C12Q 1/68, C12Q 1/02, G01N 33/15, G01N 33/50

(54) **P18ABETA RP GENE AND P18ABETA RP PROTEIN, NOVEL GENE/PROTEIN (P60TRP) INTERACTING THEREWITH TO INHIBIT CELL DEATH AND CELL DEATH PROMOTER**

(30) Priority: 07.03.2002 JP 2002061749; 28.10.2002 JP 2002312715
(71) Applicant: BF Research Institute, Inc., Osaka-shi, Osaka 532-8686 (JP)
(72) Inventor: HEESE, Klaus, Minoh-shi, Osaka 562-0025 (JP); YAMADA, Takashi, 205, Hohkumezon, Minoh-shi, Osaka 562-0032 (JP); NAGAI, Yasuo, Minoh-shi, Osaka 562-0005 (JP); SAWADA, Tohru, Ibaraki-shi, Osaka 567-0064 (JP)
(74) Representative: Walton, Seán Malcolm
(86) International application number: PCT/JP2003/002550
(87) International publication number: WO 2003/074701

(57) **Abstract**

The present invention provides a p18AβrP gene having novel functions of promoting cell death, and its product, a p18AβrP protein. The present invention also provides screening systems to which these are applied, cell-death promoting or suppressing substances obtainable by the screening system, and pharmaceutical compositions for the treatment and/or prophylaxis of diseases containing them.

Sequence Listing

<110> BF Research Institute, Inc.

<120> P18AβRP GENE AND P18AβRP PROTEIN, AND NOVEL GENE/PROTEIN (P60T
RP) SUPPRESSING CELL DEATH AND SUBSTANCES PROMOTING CELL DEATH BY INTE
RACTION WITH THE SAME

<130> 663496

<150> JP 2002-61749
<151> 2002-03-07

<150> JP 2002-312715
<151> 2002-10-28

<160> 14

<210> 1
<211> 42
<212> DNA
<213>

<400> 1
atgagtgaat ggacgaagaa aagcccctta gaatgggagg at 42

<210> 2
<211> 45
<212> DNA

<213>

<400> 2

tctgggaagc tgaaagatgg ccttgaataa gatcctgaat tcggg 45

<210> 3

<211> 1089

<212> DNA

<213> RAT

<400> 3

gcagctctgg gtatggcggc ctcaaggcag gacccagtct tcgagctcta gagcttcgct 60

ggagcgtcct cagcgcttta tgaggaaatt ggcgcaaacc ccgcggagaa tcatctagaa 120

agagtagatc tagccagctg gtaaccatga gtgaatggac gaagaaaagc cccttagaat 180

gggaggatca cgtttacaaa gaagtgagag tgatagccag tgagaaggag tataaaggat 240

ggctgctaac cacagaccca gtctctgcca acattgtcct cgtgaacttc cttgaagatg 300

gcagactatg tgtgactgga attatgggac attctgtgca gacagtggaa actgtaagcg 360

aaggggacca cagagtaaga gagaagctga tgcatctgtt cacacctgca gattgtaaag 420

ggtacagccc tgaggatctg gaaaagaaga aaaccagcct aaagaaatgg cttgagaaga 480

accacatccc tgtcactgaa gagggagaca cacaaaggac tctctgtgtg gctggggttc 540

ttactataga cccaccatat gctccagaaa attgcagcag ctctaacgag tttattctgt 600

cccgaattca ggatcttatt caaggccatc tttcagcttc ccagtgagag gccgcacgag 660

gagcacactg acttcactgt ttggttctgt attaaattct tccagtgtaa gttgattata 720

ttacaagact tcaaagcaca tgactactat gtgtatatgc gcacattttt ttttttcttt 780

ttcttttttt tcggagctgg ggaccgaacc cagggccttg cgtttgctag gcaagcgctc 840

taccactgag ctaaatcccc aaccccatat gcgtacattt taagtttttg tttaggtcaa 900

atcagaggaa gtgaaggcac cagaacagtg tcccttgtcc tgaaaagaac acaggagagt 960

caaatttgga aggagatttc cttgcatatg atttaagata aatcacccta tttgtgagac 1020

aagggtgcat tttgaatagg tgtaacaatg tgaaataaac ttgtaaattt caatataaga 1080

tattaaagt                                                    1089

<210> 4

<211> 166

<212> PRT

<213> RAT

<400> 4

Met Ser Glu Trp Thr Lys Lys Ser Pro Leu Glu Trp Glu Asp His Val

1               5                   10                  15

Tyr Lys Glu Val Arg Val Ile Ala Ser Glu Lys Glu Tyr Lys Gly Trp

            20                  25                  30

Leu Leu Thr Thr Asp Pro Val Ser Ala Asn Ile Val Leu Val Asn Phe

        35                  40                  45

Leu Glu Asp Gly Arg Leu Cys Val Thr Gly Ile Met Gly His Ser Val

    50                  55                  60

Gln Thr Val Glu Thr Val Ser Glu Gly Asp His Arg Val Arg Glu Lys

65                  70                  75                  80

Leu Met His Leu Phe Thr Pro Ala Asp Cys Lys Gly Tyr Ser Pro Glu

                85                  90                  95

Asp Leu Glu Lys Lys Lys Thr Ser Leu Lys Lys Trp Leu Glu Lys Asn

            100                 105                 110

His Ile Pro Val Thr Glu Glu Gly Asp Thr Gln Arg Thr Leu Cys Val

        115                 120                 125

Ala Gly Val Leu Thr Ile Asp Pro Pro Tyr Ala Pro Glu Asn Cys Ser

    130                 135                 140

Ser Ser Asn Glu Phe Ile Leu Ser Arg Ile Gln Asp Leu Ile Gln Gly

145                 150                 155                 160

His Leu Ser Ala Ser Gln

                165

<210> 5

<211> 2701

<212> DNA

<213> RAT

<400> 5

agttaccaag tataaagata ccagcctgga acagagaact aaggataaga ctggacaggt 60

gtatataact gtgcttcaac catgactggc tcaaagaata aggctcgggc tcaggctaaa 120

ctggaaaaga gggcaagtgc acaagccaaa gctgcagcag agagagaggc tgctaatgca 180

ggcagaggtg caggcaaaaa ccgggacaaa gggaagggta aggcaggctc taaaacagat 240

gcagtggcag aggcgaaggc gggctctaag agcaaggtag ttgctgagac aaaagaagga 300

gcaagaccag aatctaaggc tgtagcaaaa ggcacatcag atttcaacca taaggctgag 360

aacaagtacg ctagatccgc acgtaaagat aagcccagta gtgatagctg gttttgggct 420

ggagaagatt ctggtatcaa ttcctggttc tggaagggag aagaggttag taacaattct 480

gttgccaagt gtgaaaataa acctagtact agtatccagg cccgtgtgga ggagcacacg 540

cctagaacca gccacaagtc taggtcagga gctgaggaag aggaggaaga gaatgttata 600

gggaactggt tttgggaagg agatgacact ggttttgatt ctgatcctaa acctgtgttc 660

aaaatagtaa aacctcagcc agtagatgaa ataaatgaaa aggataggcc aaaggactgg 720

tccgaggtaa ctatctggcc caaagctcct gctgtaactc cagcagtgtt aggttataga 780

tctcaggact catctgaggg aaggccctct tcatatattg ttctggcctc aaatgaagag 840

gaaacttcaa caacctgtac taagaatact cgttcaagcc tccagcctat acctgagtat 900

ccatttggat ctgatccttg catacagacc ttagatgaaa ttagacagca aatcaagatc 960

agagaagaga atggcatcaa gccctttgct tgcccttgca aaatggagtg ctatttggat 1020

tctccggaat ttgaaaagct tgttaacata cttaagtcaa ctactgatcc ccttattcat 1080

aaaatagcac agattgcaat gggtatccat aaagttcatc cgtttgccca ggaattcatt 1140

aatgaagtgg gtgtggtgac gcttattgaa agcttgctca gtttttcttc ccctgaagtt 1200

agtataaaaa aggctgttat tactctgaac tcttccgggg atgacagaca acaaatggtt 1260

gaatttcatg ttaagcatat gtgtaaagaa acggtatctt tccccttgaa ctcacctggc 1320
cagcaatctg gattaaagat aatagggcag ctgacgactg agtctgtcca tcactacatt 1380
gtagttagct acttttcaga gcttttccat ttgctgtccc agggaaatcg taagactagg 1440
aatcttgttt tgaaagtatt tttgaatatg tctgaaaatc ccaaggcagc cagagatatg 1500
ataaatatga aggcattagc agcattaaaa ctcatcttta accaaaaaga ggcaaaagcc 1560
aatcttgtga gtgctgtggc catctttatt aacataaagg agcatattag aaagggctca 1620
attgtagtag ttgatcactt gagttacaat actcttactg ccatattccg tgaagttaaa 1680
gggattattg aaagaatgta aaatgaccaa gaaattgaag agaacactga acagtgtcca 1740
aaatctgatt ggctgtacat tcccaaagag ttttgcataa tattttggta attactgctc 1800
acacattttt gtcttaacat cttttacata ttattacctg tggcaggttc tagatcaaag 1860
ctggaacatt tttgatgtat caaatgaata ttacatcttg agataaaaag tttggtgatt 1920
tctatcttat ctagattagc agattttaac attttactta aggaaactga tccaactctc 1980
aaaagtacag tgtcttgagg aatagtatct gcttaggtct ggttgtggct tcaggtagca 2040
aaaagaaact aatgggcttg ggatctagtt acagaaataa ggcatatgga aataaagata 2100
tgtgatggta cttacactgt gtgtgtgtac atgatataaa catatatatg cctattgcca 2160
aatgtgtact cttgactcaa aaaggaggga ggtcactata cgctattaaa tagaagagtg 2220
atttctatat ccctctttat ccaactgcat caaacatttc actgaagaat acagaatggc 2280
cctgaagtat acaaatctat aatatattca ttttaaaatt tgcttttata actaaaattc 2340
caattttaca ctattttatc tgtcacctca aagaataaaa aggtaagcat gaaaagaata 2400
tatttttatt atcagttgct attacacact tatattggca tattttacaa cttttatttt 2460
ttatggtgat cctgtgaact agttaactca tttttttatc tcactatacc acttttacac 2520
tcattccctc ttttaggtga tagccttcac agcacagaga ctgtttaggt tatagtagta 2580
gtttaaattt ctgatgaata atgtccccta ccaggttaac agctattggc ttccttcaca 2640
ttcaggttga ctagtcagtt atctggctag atgccagatg gagtgtctga aatgagtact 2700
t 2701

<210> 6
<211> 539
<212> PRT

<213> RAT

<400> 6

Met Thr Gly Ser Lys Asn Lys Ala Arg Ala Gln Ala Lys Leu Glu Lys
1               5                   10                  15

Arg Ala Ser Ala Gln Ala Lys Ala Ala Ala Glu Arg Glu Ala Ala Asn
            20                  25                  30

Ala Gly Arg Gly Ala Gly Lys Asn Arg Asp Lys Gly Lys Gly Lys Ala
        35                  40                  45

Gly Ser Lys Thr Asp Ala Val Ala Glu Ala Lys Ala Gly Ser Lys Ser
    50                  55                  60

Lys Val Val Ala Glu Thr Lys Glu Gly Ala Arg Pro Glu Ser Lys Ala
65                  70                  75                  80

Val Ala Lys Gly Thr Ser Asp Phe Asn His Lys Ala Glu Asn Lys Tyr
                85                  90                  95

Ala Arg Ser Ala Arg Lys Asp Lys Pro Ser Ser Asp Ser Trp Phe Trp
            100                 105                 110

Ala Gly Glu Asp Ser Gly Ile Asn Ser Trp Phe Trp Lys Gly Glu Glu
        115                 120                 125

Val Ser Asn Asn Ser Val Ala Lys Cys Glu Asn Lys Pro Ser Thr Ser
    130                 135                 140

Ile Gln Ala Arg Val Glu Glu His Thr Pro Arg Thr Ser His Lys Ser
145                 150                 155                 160

Arg Ser Gly Ala Glu Glu Glu Glu Glu Glu Asn Val Ile Gly Asn Trp
                165                 170                 175

Phe Trp Glu Gly Asp Asp Thr Gly Phe Asp Ser Asp Pro Lys Pro Val
            180                 185                 190

Phe Lys Ile Val Lys Pro Gln Pro Val Asp Glu Ile Asn Glu Lys Asp
        195                 200                 205

Arg Pro Lys Asp Trp Ser Glu Val Thr Ile Trp Pro Lys Ala Pro Ala
    210                 215                 220
Val Thr Pro Ala Val Leu Gly Tyr Arg Ser Gln Asp Ser Ser Glu Gly
225                 230                 235                 240
Arg Pro Ser Ser Tyr Ile Val Leu Ala Ser Asn Glu Glu Glu Thr Ser
                245                 250                 255
Thr Thr Cys Thr Lys Asn Thr Arg Ser Ser Leu Gln Pro Ile Pro Glu
            260                 265                 270
Tyr Pro Phe Gly Ser Asp Pro Cys Ile Gln Thr Leu Asp Glu Ile Arg
        275                 280                 285
Gln Gln Ile Lys Ile Arg Glu Glu Asn Gly Ile Lys Pro Phe Ala Cys
    290                 295                 300
Pro Cys Lys Met Glu Cys Tyr Leu Asp Ser Pro Glu Phe Glu Lys Leu
305                 310                 315                 320
Val Asn Ile Leu Lys Ser Thr Thr Asp Pro Leu Ile His Lys Ile Ala
                325                 330                 335
Gln Ile Ala Met Gly Ile His Lys Val His Pro Phe Ala Gln Glu Phe
            340                 345                 350
Ile Asn Glu Val Gly Val Val Thr Leu Ile Glu Ser Leu Leu Ser Phe
        355                 360                 365
Ser Ser Pro Glu Val Ser Ile Lys Lys Ala Val Ile Thr Leu Asn Ser
    370                 375                 380
Ser Gly Asp Asp Arg Gln Gln Met Val Glu Phe His Val Lys His Met
385                 390                 395                 400
Cys Lys Glu Thr Val Ser Phe Pro Leu Asn Ser Pro Gly Gln Gln Ser
                405                 410                 415
Gly Leu Lys Ile Ile Gly Gln Leu Thr Thr Glu Ser Val His His Tyr
            420                 425                 430
Ile Val Val Ser Tyr Phe Ser Glu Leu Phe His Leu Leu Ser Gln Gly

        435                 440                 445
Asn Arg Lys Thr Arg Asn Leu Val Leu Lys Val Phe Leu Asn Met Ser
    450                 455                 460
Glu Asn Pro Lys Ala Ala Arg Asp Met Ile Asn Met Lys Ala Leu Ala
465                 470                 475                 480
Ala Leu Lys Leu Ile Phe Asn Gln Lys Glu Ala Lys Ala Asn Leu Val
                485                 490                 495
Ser Ala Val Ala Ile Phe Ile Asn Ile Lys Glu His Ile Arg Lys Gly
            500                 505                 510
Ser Ile Val Val Val Asp His Leu Ser Tyr Asn Thr Leu Thr Ala Ile
        515                 520                 525
Phe Arg Glu Val Lys Gly Ile Ile Glu Arg Met
    530                 535

<210> 7
<211> 1647
<212> DNA
<213> HUMAN

<400> 7
accatggctg ggactaagaa taagacaaga gcccaggcca aaactgaaaa aaaggctgct 60
atacaagcta aagctggagc agagagggag gctactggtg ttgttaggcc tgtagccaag 120
accagggcca aagcaaaagc caagacaggg tctaagacag atgcagtagc agagatgaag 180
gcagtgtcta agaacaaggt tgttgctgag acgaaggaag gagctctgtc agagcctaag 240
actctgggca aagccatggg agatttcact cccaaggctg ggaatgagtc caccagctcc 300
acatgtaaaa atgaggctgg tactgatgcc tggttctggg ctggggaaga ggccactatc 360
aattcctggt tctggaatgg agaagaggct ggtaatagtt tcagcactaa gaatgataaa 420
cctgaaattg gtgcccaggt ctgtgctgag gagttggaac ctgcggctgg ggccgattgc 480
aaacctaggt caggggctga ggaggaggag gaagagaatg ttattgggaa ctggttttgg 540

gaaggagatg atactagttt tgaccctaat cctaaacctg tgagcaggat agttaagcct 600
cagcctgtgt atgaaattaa tgaaaaaaat aggcccaagg actggtctga ggtaactatc 660
tggcccaatg cccctgctgt aactccagct gtgttaggat ttagatccca ggcaccatct 720
gaggcaagtc ctccttcata tattgttctg gcctccgctg aagaaaatgc ctgttctttg 780
cctgtggcaa cagcttgccg cccttctagg aacactcgct catgctcaca gcctatccct 840
gagtgtcgtt ttgattctga cccctgcatc cagaccatag atgagattag acgtcaaatc 900
aggatcaggg aggtaaatgg gattaagcca tttgcttgtc cttgcaaaat ggaatgctat 960
atggattctg aggaatttga aaaacttgtt agcttactta agtcaactac tgatcctctt 1020
attcataaaa tagcacggat tgcaatgggt gtccataatg ttcacccatt tgcccaagag 1080
tttattaacg aagtaggtgt agtgacactt attgaaagct tgctcagttt tccttcccct 1140
gaaatgagaa aaaagactgt aattactctg aatcctcctt ctggggatga aagacaacgc 1200
aaaattgaat tacatgttaa gcatatgtgt aaagaaacca tgtcatttcc tttgaactca 1260
ccgggacagc aatctggatt aaagatacta ggacaactga ctactgattt tgtccatcac 1320
tacattgttg ccaattactt ttcagagctt ttccatttgc tgtcctcagg aaattgcaaa 1380
accagaaatc ttgttttgaa actactttta aatatgtctg aaaatccaac tgcagccaga 1440
gacatgatca atatgaaggc attggcagca ttaaaactca tctttaacca gaaagaggca 1500
aaagccaatc ttgttagtgg tgtggccata tttattaaca taaaggagca tatcagaaaa 1560
ggctcaattg tagttgttga tcacttgagt tataatacac tcatggccat tttcagggaa 1620
gttaaagaga ttattgaaac aatgtag 1647

<210> 8
<211> 547
<212> PRT
<213> HUMAN

<400> 8
Met Ala Gly Thr Lys Asn Lys Thr Arg Ala Gln Ala Lys Thr Glu Lys
1               5                   10                  15
Lys Ala Ala Ile Gln Ala Lys Ala Gly Ala Glu Arg Glu Ala Thr Gly

            20                  25                  30
Val Val Arg Pro Val Ala Lys Thr Arg Ala Lys Ala Lys Ala Lys Thr
        35                  40                  45
Gly Ser Lys Thr Asp Ala Val Ala Glu Met Lys Ala Val Ser Lys Asn
    50                  55                  60
Lys Val Val Ala Glu Thr Lys Glu Gly Ala Leu Ser Glu Pro Lys Thr
65                  70                  75                  80
Leu Gly Lys Ala Met Gly Asp Phe Thr Pro Lys Ala Gly Asn Glu Ser
                85                  90                  95
Thr Ser Ser Thr Cys Lys Asn Glu Ala Gly Thr Asp Ala Trp Phe Trp
            100                 105                 110
Ala Gly Glu Glu Ala Thr Ile Asn Ser Trp Phe Trp Asn Gly Glu Glu
        115                 120                 125
Ala Gly Asn Ser Phe Ser Thr Lys Asn Asp Lys Pro Glu Ile Gly Ala
    130                 135                 140
Gln Val Cys Ala Glu Glu Leu Glu Pro Ala Ala Gly Ala Asp Cys Lys
145                 150                 155                 160
Pro Arg Ser Gly Ala Glu Glu Glu Glu Glu Glu Asn Val Ile Gly Asn
                165                 170                 175
Trp Phe Trp Glu Gly Asp Asp Thr Ser Phe Asp Pro Asn Pro Lys Pro
            180                 185                 190
Val Ser Arg Ile Val Lys Pro Gln Pro Val Tyr Glu Ile Asn Glu Lys
        195                 200                 205
Asn Arg Pro Lys Asp Trp Ser Glu Val Thr Ile Trp Pro Asn Ala Pro
    210                 215                 220
Ala Val Thr Pro Ala Val Leu Gly Phe Arg Ser Gln Ala Pro Ser Glu
225                 230                 235                 240
Ala Ser Pro Pro Ser Tyr Ile Val Leu Ala Ser Ala Glu Glu Asn Ala
                245                 250                 255

Cys Ser Leu Pro Val Ala Thr Ala Cys Arg Pro Ser Arg Asn Thr Arg
            260                 265                 270
Ser Cys Ser Gln Pro Ile Pro Glu Cys Arg Phe Asp Ser Asp Pro Cys
        275                 280                 285
Ile Gln Thr Ile Asp Glu Ile Arg Arg Gln Ile Arg Ile Arg Glu Val
    290                 295                 300
Asn Gly Ile Lys Pro Phe Ala Cys Pro Cys Lys Met Glu Cys Tyr Met
305                 310                 315                 320
Asp Ser Glu Glu Phe Glu Lys Leu Val Ser Leu Leu Lys Ser Thr Thr
                325                 330                 335
Asp Pro Leu Ile His Lys Ile Ala Arg Ile Ala Met Gly Val His Asn
            340                 345                 350
Val His Pro Phe Ala Gln Glu Phe Ile Asn Glu Val Gly Val Val Thr
        355                 360                 365
Leu Ile Glu Ser Leu Leu Ser Phe Pro Ser Pro Glu Met Arg Lys Lys
    370                 375                 380
Thr Val Ile Thr Leu Asn Pro Pro Ser Gly Asp Glu Arg Gln Arg Lys
385                 390                 395                 400
Ile Glu Leu His Val Lys His Met Cys Lys Glu Thr Met Ser Phe Pro
                405                 410                 415
Leu Asn Ser Pro Gly Gln Gln Ser Gly Leu Lys Ile Leu Gly Gln Leu
            420                 425                 430
Thr Thr Asp Phe Val His His Tyr Ile Val Ala Asn Tyr Phe Ser Glu
        435                 440                 445
Leu Phe His Leu Leu Ser Ser Gly Asn Cys Lys Thr Arg Asn Leu Val
    450                 455                 460
Leu Lys Leu Leu Leu Asn Met Ser Glu Asn Pro Thr Ala Ala Arg Asp
465                 470                 475                 480
Met Ile Asn Met Lys Ala Leu Ala Ala Leu Lys Leu Ile Phe Asn Gln

                485                 490                 495
Lys Glu Ala Lys Ala Asn Leu Val Ser Gly Val Ala Ile Phe Ile Asn
            500                 505                 510
Ile Lys Glu His Ile Arg Lys Gly Ser Ile Val Val Val Asp His Leu
        515                 520                 525
Ser Tyr Asn Thr Leu Met Ala Ile Phe Arg Glu Val Lys Glu Ile Ile
    530                 535                 540
Glu Thr Met
545

<210> 9
<211> 33
<212> DNA
<213>

<400> 9
gcgtaatacg actcactata gggaattcga cgt 33

<210> 10
<211> 33
<212> DNA
<213>

<400> 10
cgcgacgtac gatttaaatt aaccctcact aaa 33

<210> 11
<211> 42
<212> DNA

<213>

<400> 11
atgactggct caaagaataa ggctcgggct caggctaaac tg          42

<210> 12
<211> 45
<212> DNA
<213>

<400> 12
ttacattctt tcaataatcc ctttaacttc acggaatatg gcagt       45

<210> 13
<211>
<212> DNA
<213>

<400> 13
atggctggga ctaagaataa gacaagagcc caggccaaaa c           41

<210> 14
<211>
<212> DNA
<213>

<400> 14
cattgtttca ataatctctt taacttccct gaaaatggcc atgag       45

## Description

### TECHNICAL FIELD

The present invention relates to a novel gene, p18AβrP, whose expression is increased in oligodendrocytes by amyloid-β protein (hereinafter abbreviated to Aβ). The present gene and its product, a p18AβrP protein, have novel functions of suppressing the promotion of neurite elongation and the sustaining of survival by neurotrophic factors to promote cell death, by interacting with the heat shock protein Hsp 70 and the tumor suppressor protein Tid-1. The present invention also relates to screening systems to which these events are applied, a cell-death suppressing gene and protein (p60TRP) identified using the screening system, and cell-death promoting substances and their genes. In addition, the present invention relates to diagnosis, treatment, and prophylaxis of diseases associated with cell death, employing these cell-death suppressing or promoting substances.

### BACKGROUND ART

At present, the cause of Alzheimer's disease is unknown. However, there are reported, as its pathological characteristics, senile plaques, neurofibrillary tangles, remarkable encephalatrophies of cerebral cortex and hippocampus by cell death, and the like. Hyman et al. (Science, 225, 1168 (1984)) and Braak et al . (Acta Neuropathol., 82, 239 (1991)), respectively, found specific degenerations in the area of and around entorhinal cortex as early pathological changes in Alzheimer's disease, and Braak et al. reported that in entorhinal cortex, their causes were suspected to be due to the result of the degeneration of oligodendrocytes supplying nutriments to nerve cells (Alzheimer's Res., 3, 235 (1997)). However, any investigation to seek their ascertainment has not been made yet.

### DISCLOSURE OF THE INVENTION

In view of these circumstances, the present inventors have intensively studied. As a result, they observed cell death when used rat oligodendrocytes and added Aβ, the main component of senile plaques present in the brain affected with Alzheimer's disease. Screening of genes involved in this cell death resulted in finding a novel gene p18AβrP. In addition, examining of functions of this gene gave such a result that in cells in which this gene was expressed, the induction of cell differentiation triggered by neurotrophic factors was inhibited to cause cell death. Additionally, it turned out that the present protein, i.e. the transcription product of this gene, has novel functions of suppressing the elongation and branching of neurites and the sustaining of survival to promote cell death by interacting with the heat shock protein Hsp 70 and/or the tumor suppressor protein Tid-1. Furthermore, screening of genes/proteins suppressing this cell death resulted in finding a novel gene/protein p60TRP.

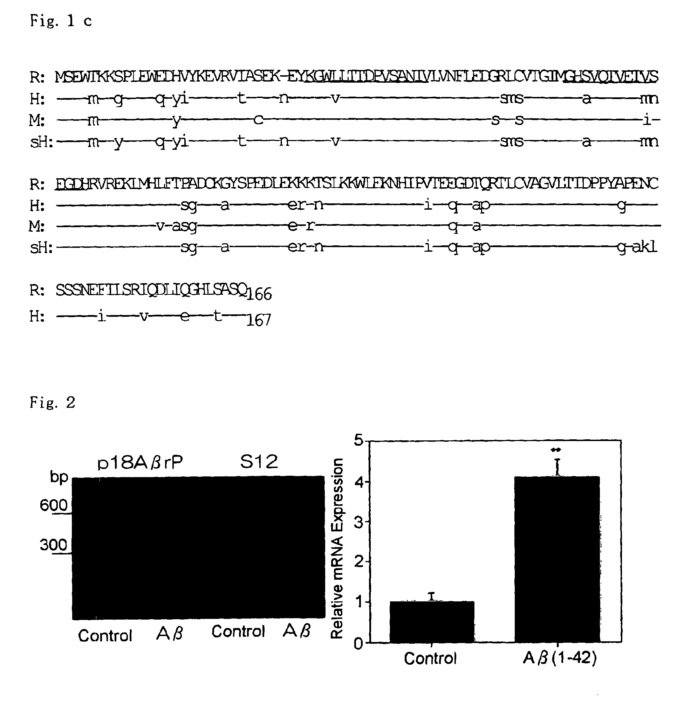

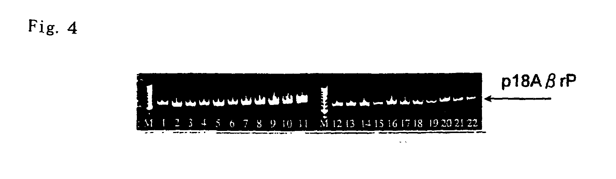

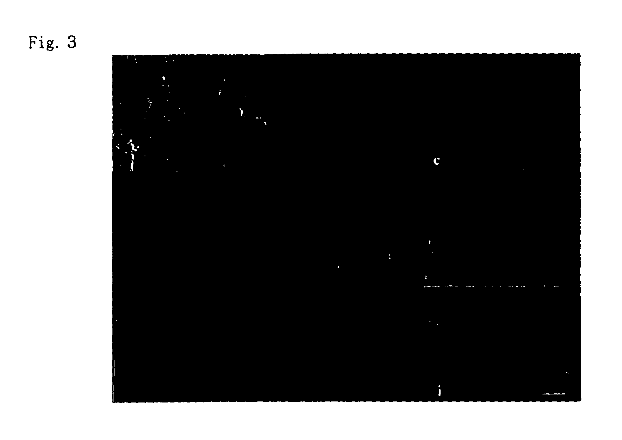

The present inventors added to rat oligodendrocyte CG-4 cells Aβ1-42 (containing amino acids 1 to 42 of β-amyloid protein; treatment at 10 µg/ml and at 37°C for 60 hours), as an example of β-amyloid protein (Aβ) considered to be a neurotoxin of Alzheimer's disease, with the result that cell death was observed in about 50% of the cells (Brain Aging, 2, 30 (2002)). Cell death like this was also identified with other Aβ peptides such as Aβ25-35 (containing amino acids 25 to 35 of β-amyloid protein), Aβ1-40 (containing amino acids 1 to 40 of β-amyloid protein), or Aβ1-43 (containing amino acids 1 to 43 of β-amyloid protein) (see, for example, Cell. Mol. Life. Sci., 57, 705 (2000); J. Neurosci., 21, 9235 (2001)), and therefore it is believed that properties causing cell death are universal to Aβ peptides. Thus, the present inventors conducted the screening of genes involved in cell death induced by Aβ, with the result that increased expression of the present gene was observed (see Examples and Fig. 2). The present cDNA displays homologies to already found genes of human (J. Biol. Chem., 277, 7540 (2002)) and mouse (Gene Data Bank AK013396, AK018385, AK020147, AK011454; Hayashizaki, Y. et al., 2000), but is a novel gene and has differences in the coded amino acid sequence from human and mouse sequences. In addition, it turned out that the p18AβrP protein has novel functions of suppressing a neurite-elongating effect of neurotrophic factors, to induce cell death, as a result of interacting with the heat shock protein Hsp 70 and the tumor suppressor protein Tid-1 (see Examples and Fig. 3). Furthermore, screening of factors suppressing this cell death observed in cells expressing the present gene resulted in finding a novel gene/protein (p60TRP).

Therefore, the present invention provides DNAs, proteins, substances, a vector, transformants, pharmaceutical compositions, and a kit according to (1) to (31) below:
(1) a p18AβrP cDNA comprising the base sequence of nucleotides 147 to 647 of SEQ ID NO:3;
(2) an mRNA which is complementary to the cDNA according to (1);
(3) a p18AβrP protein having the amino acid sequence of SEQ ID NO:4;
(4) a DNA coding for a p18AβrP protein which has one or more amino acids inserted, deleted, or substituted in the protein according to (3) and possesses properties of:
   a) increasing the expression by Aβ,
   b) interacting with Hsp70 and/or Tid-1, and
   c) inhibiting the cell differentiation;
(5) a DNA which is hybridizable under stringent conditions with the DNA according to (1) and codes for a p18AβrP protein possessing properties of:
   a) increasing the expression by Aβ,
   b) interacting with Hsp70 and/or Tid-1, and
   c) inhibiting the cell differentiation;
(6) a p18AβrP protein coded by the DNA according to (4) or (5);
(7) a vector containing the DNA according to any one of (1) , (4), and (5) ;
(8) a transformant which has undergone gene transfer by means of the vector according to (7);
(9) a method of screening cell-death promoting or suppressing substances, which comprises contacting a cell expressing p18AβrP with a test substance in the presence of a differentiation inducing factor, and determining the suppression or promotion of cell death;
(10) a substance interacting with p18AβrP to suppress cell death;
(11) a substance interacting with p18AβrP to suppress cell death, wherein the substance is found by the cell assay system according to (9);
(12) a substance according to (10) or (11), which suppresses cell death by affecting the interaction of p18AβrP with Hsp70 and/or Tid-1;
(13) a cDNA comprising the base sequence of nucleotides 82 to 1701 of SEQ ID NO:5;
(14) an mRNA which is complementary to the cDNA according to (13) ;
(15) a protein having the amino acid sequence of SEQ ID NO:6;
(16) a DNA coding for a protein which has one or more amino acids inserted, deleted, or substituted in the protein according to (15) and possesses a cell-death suppressing effect similar to that of the protein according to (15);
(17) a DNA which is hybridizable under stringent conditions with the DNA according to (13) and codes for a protein possessing a cell-death suppressing effect similar to that of the protein according to (15);
(18) a protein coded by the DNA according to (16) or (17) and possessing a cell-death suppressing effect similar to that of the protein according to (15);
(19) a protein according to (15), which is p60TRP, or its variant protein possessing a cell-death suppressing effect similar to that of p60TRP;
(20) a vector containing the DNA according to any one of (13), (16) and (17);
(21) a transformant which has undergone gene transfer by means of the vector according to (20);
(22) a pharmaceutical composition for the treatment of diseases associated with cell death, which contains a protein, DNA, vector, or transformant according to any one of (10) to (21);
(23) a substance interacting with p18AβrP to promote cell death;
(24) a substance interacting with p18AβrP to promote cell death, which the substance is found by the cell assay system according to (9);
(25) a substance according to (23) or (24), which promotes cell death by affecting the interaction of p18AβrP with Hsp70 and/or Tid-1;
(26) a pharmaceutical composition for the treatment and/or prophylaxis of diseases caused by cell hyperproliferation, which contains a substance according to any one of (23) to (25);
(27) a method for the diagnosis of diseases associated with cell death, characterized by determining the level of expression of the p18AβrP gene or p18AβrP protein in cells or tissues obtained from a subject;
(28) a kit for the diagnosis of diseases associated with cell death, characterized by determining the level of expression of the p18AβrP gene or p18AβrP protein in cells or tissues obtained from a subject;
(29) a substance which enhances a cell-death suppressing effect of p60TRP by interacting with p60TRP to inhibit the cell-death signal via p18AβrP;
(30) a substance which attenuates a cell-death suppressing effect of p60TRP by interacting with p60TRP to inhibit the suppression of the cell-death signal via p18AβrP; and
(31) a pharmaceutical composition according to (22), which further contains a substance according to (29) enhancing a cell-death suppressing effect of p60TRP.

### BRIEF DESRIPTION OF THE DRAWINGS

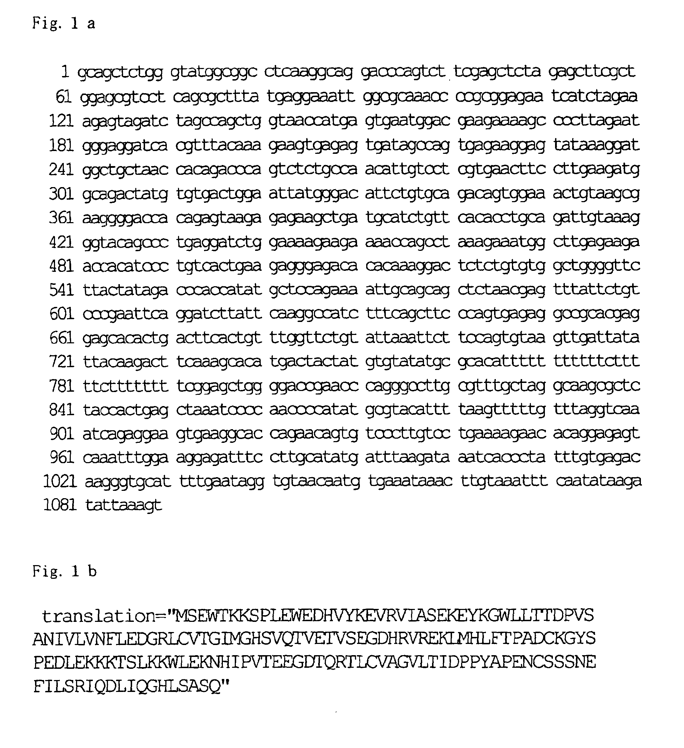

Fig. 1 shows the base sequence (Fig. 1a) and the amino acid sequence (Fig. 1b) of a p18AβrP cDNA. Fig. 1c shows a comparison of the amino acid sequences of the rat p18AβrP protein of the present invention (R), and human (H) and mouse (M) homologous proteins, and a human short homologous protein (sH). The amino acid sequences underlined in Fig. 1c indicate putative nNOS PDZ domains.

Fig. 2 (left panel) shows a Southern blot of rat CG4 oligodendrocyte lysates after the addition of Aβ1-42 (10 µg/ml) at 37°C for 60 hours: lane 1, control; lane 2, Aβ treatment (Aβ). Fig. 2 (right panel) shows quantifications of the p18AβrP mRNA transcript. Values are expressed as densitometric ratio of PCR products of p18AβrP and S12 ± standard error in six individual experiments (** P < 0.01, significance level versus the control group).

Fig. 3 shows the neurite elongation in cells expressing or not expressing p18AβrP after the addition of NGF. At 120 hours after NGF was added, p18AβrP-positive cells showed no neurite elongation (indicated by arrow), whereas cells without expression clearly showed neurite elongation. The scale bar in Panel i corresponds to 25 µm. The scale bar of this length corresponds to 75 µm in Panel a, and 50 µm in Panels b to h. The p18AβrP-positive cells did not survive more than 2 weeks after its expression, and were killed.

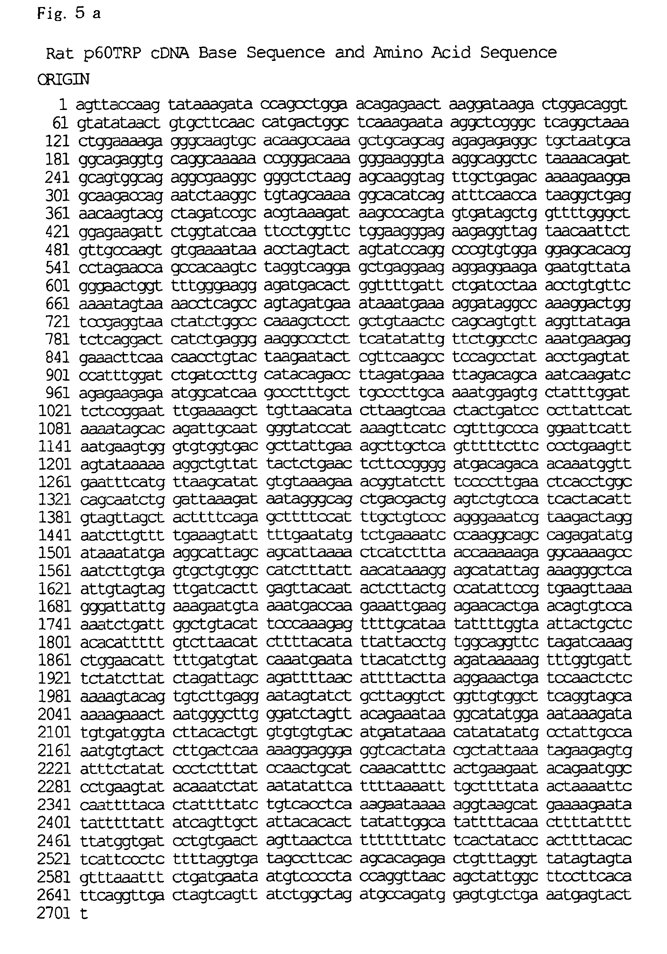

Fig. 4 shows the result of investigating the expression pattern of p18AβrP. It was shown that its mRNA was expressed in all of the 22 tissues examined. The expression was demonstrated in the following tissues: 1, brain; 2, heart; 3, kidney; 4, spleen; 5, liver; 6, colon; 7, liver; 8, small intestine; 9, muscle; 10, stomach; 11, testis; 12, salivary gland; 13, throid; 14, adrenal; 15, pancreas; 16, ovary; 17, uterus; 18, prostate; 19, skin; 20, leukocyte; 21, bone marrow; and 22, fetus brain.

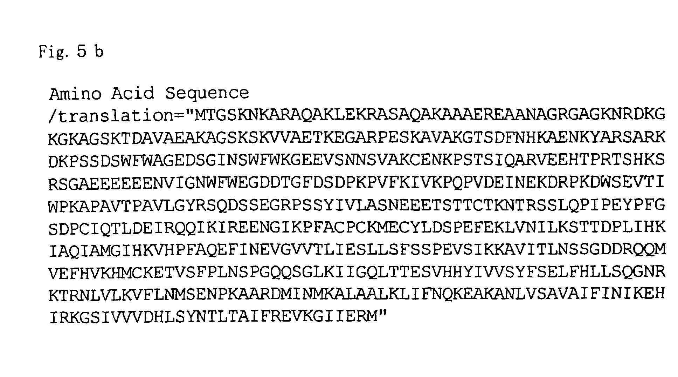

Fig. 5a shows the nucleotide sequence of a rat p60TRP cDNA and Fig. 5b shows the amino acid sequence of a rat p60TRP protein.

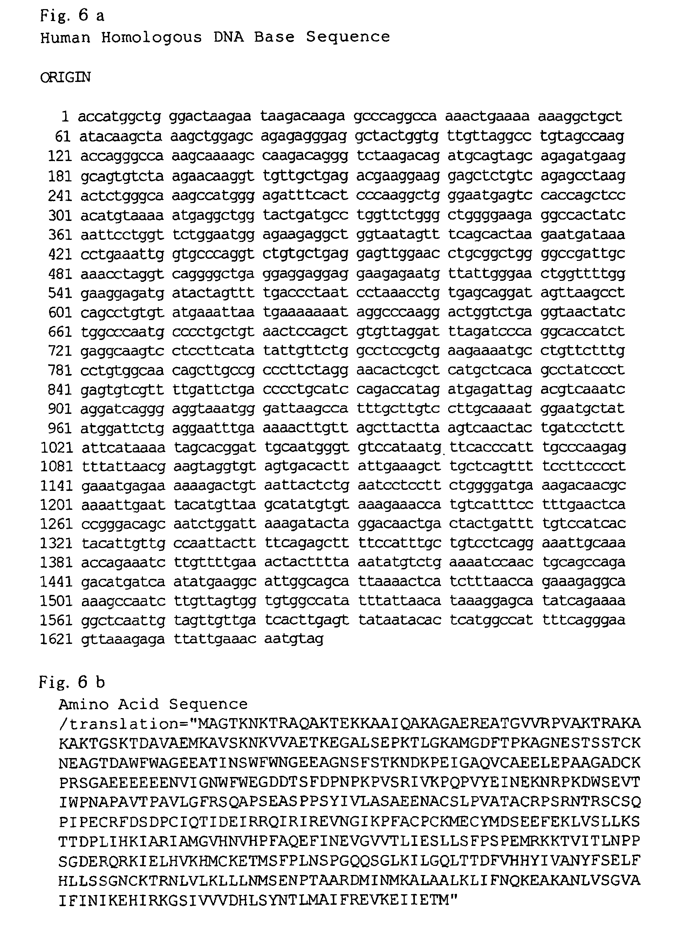

Fig. 6a shows the nucleotide sequence of a human homologous p60TRP cDNA and Fig. 6b shows the amino acid sequence of a human p60TRP protein.

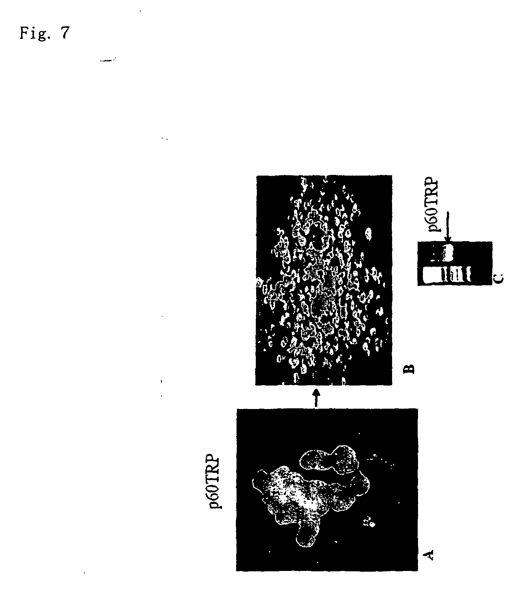

Fig. 7 shows the presence of p60TRP and the result of RT-PCR in surviving cells (PC12 cells). Panel A shows surviving colonies, Panel B shows surviving colonies cultured for 3 days, and Panel C shows electrophoresis on agarose gel of RT-PCR products.

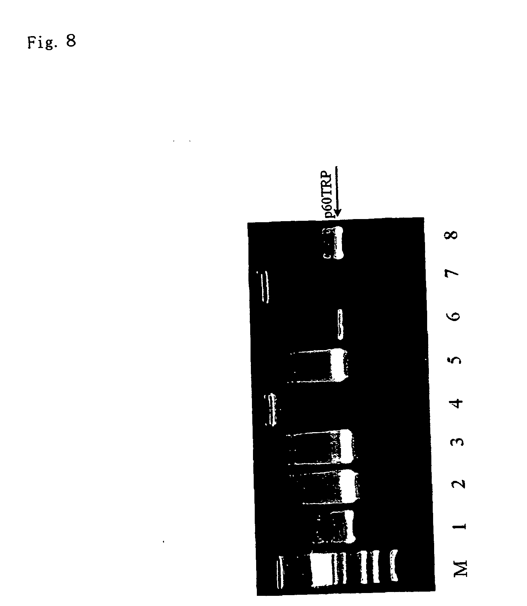

Fig. 8 shows a semi-quantification of mRNA expression by RT-PCR, representing the tissue-specific expression of p60TRP: lane M, markers; lane 1, heart; lane 2, brain; lane 3, kidney; lane 4, liver; lane 5, spleen; lane 6, pancreas; lane 7, lung; and lane 8, skeletal muscle.

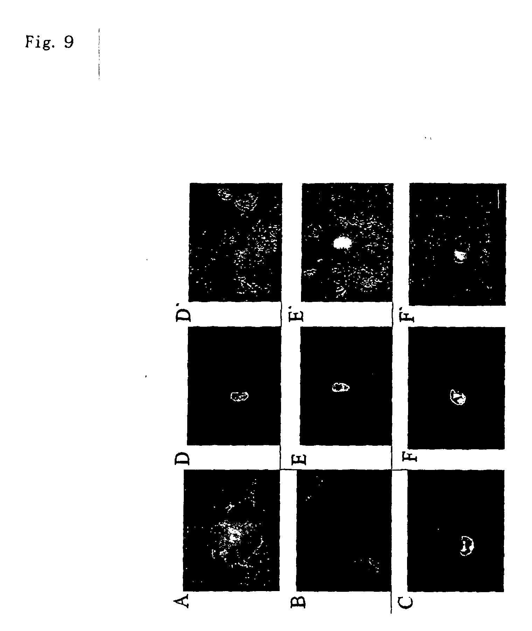

Fig. 9 shows fluorescence images of p60TRP-GFP and p60TRP-DsRedI in CHO cells. Each Panel has the same magnification. Panel A represents p60TRP-CT-GFP, and the others (B to F') represent p60TRP-CT-DsRed1. Panel B shows the presence of p60TRP in the cytoplasm, and Panel C shows the presence of p60TRP in the nucleus. Panels D and D' , E and E', and F and F' , respectively, show its presence in the nucleus, and images of the cell periphery also can be seen by the Panels marked with prime. The length of the scale bar in Panel F' corresponds to 50 µm.

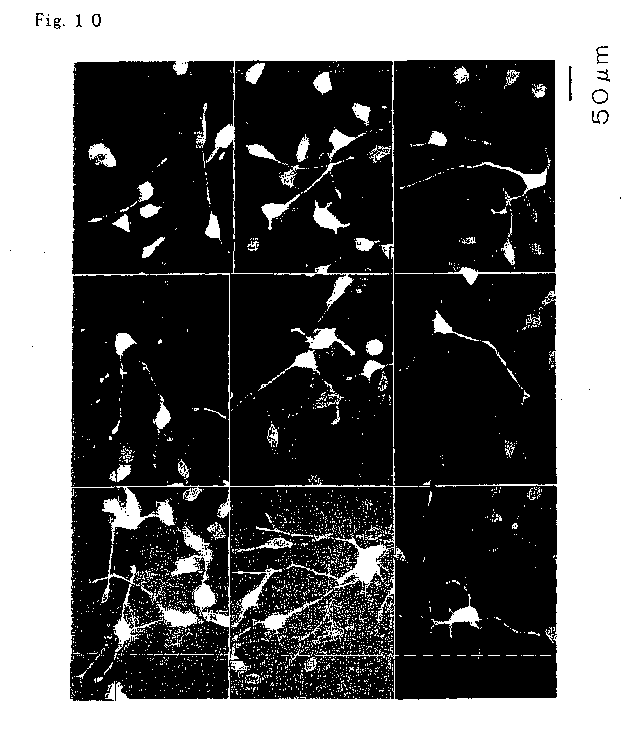

Fig. 10 shows effects on PC12 cells by expressing p60TRP. Fluorescence microscope images are shown which were taken after p60TRP was co-expressed with GFP by means of p60TRP-IRES-GFP and NGF (50 ng/ml) was added 24 hours after the expression, followed by culturing for additional 120 hours. Cells expressing p60TRP can be distinguished by fluorescence emitted from GFP. In PC 12 cells, p60TRP did not affect NGF-induced neurite elongation. The scale bar corresponds to 50 µm. Each Panel has the same magnification.

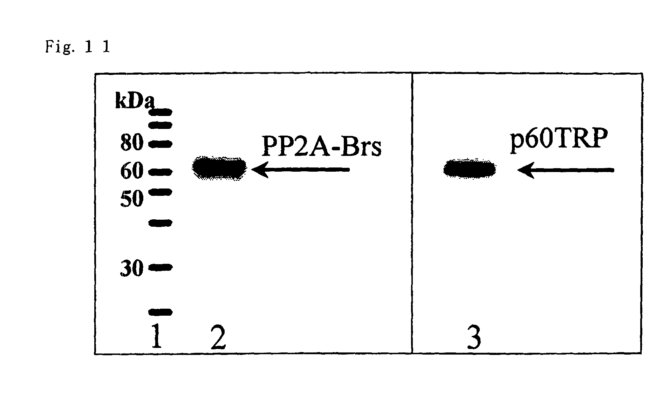

Fig. 11 shows the results of Western blot analysis of PP2A. Lane 1 indicates molecular weight markers, and lane 2 indicates a protein precipitated with a polyclonal goat anti-PP2A antibody when employing PC12 cells into which p60TRP-IRES-GFP has been introduced. Lane 3 indicates a protein precipitated with a polyclonal rabbit anti-p60TRP antibody, with a single band detected at about 60 kDa.

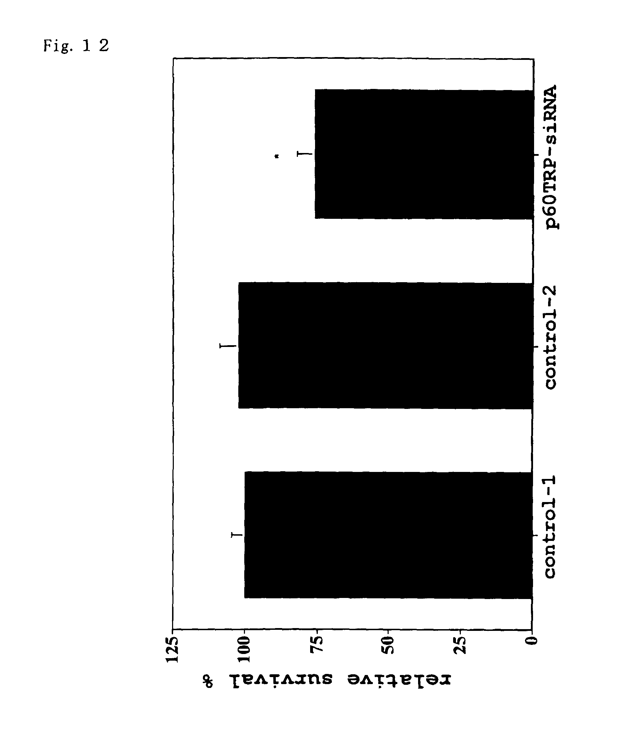

Fig. 12 shows percentages of cell survival in PC12 cells by p60TRP gene knock-out. Each percent survival is plotted as means ± standard error in 8 independent experiments. Relative to a percent survival of 100% in control-1, no change could be seen in control-2 expressing only GFP, having 102%, whereas the percent survival was significantly reduced to 76% with p60TRP knock-outed p60TRP-siRNA. Significance level: * p < 0.05 (versus control).

### DETAILED DESCRIPTION OF THE INVENTION

The present invention will be further described in detail below.

In one embodiment, the present invention relates to a p18AβrP cDNA comprising the base sequence of nucleotides 147 to 647 of SEQ ID NO: 3. The present invention also relates to an mRNA which is complementary to the cDNA described above. The above-described cDNA codes for a p18AβrP protein as shown in SEQ ID NO:4, and therefore the p18AβrP protein of the present invention has the amino acid sequence of SEQ ID NO:4. The above-described cDNA can be typically obtained by methods employing, for example, RT-PCR procedures, cDNA differential procedures, and others, as described in the section Methods in Examples, which methods are known to those skilled in the art.

In another embodiment, therefore, the present invention relates to a p18AβrP protein having the amino acid sequence of SEQ ID NO:4. Analysis of amino acid sequences of proteins can be performed, for example, by methods described in the section Methods in Examples. This protein is coded by the base sequence of nucleotides 147 to 647 of SEQ ID NO:3. This protein possesses properties of:
a) increasing the expression by Aβ,
b) interacting with Hsp70 and/or Tid-1, and
c) inhibiting the cell differentiation.

With respect to the property a), as mentioned above, the present inventors identified cell death when conducted experiments by conducted employing rat oligodendrocytes, and adding Aβ protein, the main component of senile plaques, cleaved from its precursor protein APP to the cells, and thus screening genes involved in this cell death resulting in finding a novel gene p18AβrP was found. That is to say, the expression of the p18AβrP protein is increased by Aβ. The increased expression can be observed at both mRNA and protein levels: the increase in mRNA amount can be examined, for example, by RT-PCR, complimentary hybridization, RNase protection analysis, and the like, and the increase in protein expression can be examined, for example, by Western blotting, radioimmunoassay, fluorescent antibody methods, immunological antibody methods, and the like. With respect to the property b), the present inventors revealed novel functions, such that p18AβrP protein suppresses the elongation and branching of neurites and the sustaining of survival by differentiation inducing factors to promote cell death through the interaction with the heat shock protein Hsp70 and/or the tumor suppressor protein Tid-1 (see Examples), as mentioned above. Furthermore, with respect to the property c), the present inventors found that cells expressing this gene inhibited cell differentiation induced by neurotrophic factors and gave rise to cell death (see Examples). Thus, the activity of this protein can be determined by examining the degree of such differentiation inhibition and cell death (for example, the percentage of cells subjected to cell death, the time to cell death) . An example of methods for measuring activities is a method comprising introducing a gene coding for the p18AβrP protein into an appropriate vector, using it to transform a cell, preferably nerve cell, culturing the transformed cell under the action of a differentiation inducing factor (for example, NGF) , and comparing the cell death to that of an untransformed cell cultured under the same condition (control). In order to evaluate whether cell death has been caused, a microscopic method is commonly used. When cell death takes place, there are observed, for example, cell condensation, vacuolation, surface smoothing, fragmentation of the cell and the nucleus, and the like.

The p18AβrP protein of the present invention also includes variants of the protein of SEQ ID NO: 4. That is, any protein having the above-described properties a), b) , and c), even though one or more amino acids are inserted, deleted, or substituted in the protein of SEQ ID NO:4 is within the p18AβrP protein of the present invention. Any DNA coding for such a protein is also included in the present invention. Insertion, deletion, or substitution of one or more amino acids can be made by known methods, such as, for example, site-directed mutagenesis and PCR. Thus, even though a protein has one or more amino acids inserted, deleted, or substituted, such a protein retains the above-described properties a), b), and c), and also falls within the p18AβrP protein of the present invention.

The present invention further relates to a DNA which is hybridizable under stringent conditions with the cDNA comprising the base sequence of nucleotides 147 to 647 of SEQ ID NO:3. This DNA codes for a p18AβrP protein possessing the above-described properties a), b), and c). Stringent conditions are well known to those skilled in the art and described in many textbooks and the literature, for example. Stringent conditions are exemplified by conditions allowing hybridization at 42°C in a solution containing 6X SSC, 5X Denhardt's reagent, 0.5% SDS, and 100 µg/ml salmon sperm DNA.

The present invention further relates to a DNA having a nucleotide sequence homology of at least 60% or more, preferably 70% or more, more preferably 80% or more, and most preferably 90% or more and less than 100%, based on a cDNA comprising the base sequence of nucleotides 147 to 647 of SEQ ID NO:3.

A protein coded by any one of the DNAs mentioned above is also included in the p18AβrP protein of the present invention.

In a further embodiment, the present invention relates to a vector containing any one of the DNAs described above coding for the p18AβrP protein. Vectors which may be used for introducing any one of the above-described DNAs coding for the p18AβrP protein to transform a nerve cell utilize a variety of vectors described in the literature or commercial available vectors, for example, pVgRXR, pIND, pIND/V5-His, pIND/GFP, pcDNA3.1 or pcDNA3.1/myc or pcDNA3.1/His or pcDNA3.1/V5-His or pcDNA3.1-CT-GFP-TOPO®, or virus vectors such as LNCX2. Methods for transformation are known, such as, calcium phosphate methods, Super Fector reagent methods, and lipid-mediated methods employing LipofectAMINE reagent or the like. Cells to be used for screening may utilize any kind of cells, preferably nerve cells. Examples of preferred nerve cells include PC12 cell, NB2a, Neuro-2A, B104, SHSY-5Y, primary culture nerve cell, and the like. The above-described vector, cell transformation, and conditions for culturing cells are determined depending upon individual cells, and are within the knowledge of those skilled in the art or can be readily determined. Such transformants are included in the present invention. It is desirable to attach a detectable tag to a p18AβrP protein to be expressed, so that the expression of the p18AβrP protein can be detected with ease. The tag can be attached at the DNA level: for example, green fluorescent protein DNA, red fluorescent protein DNA, myc gene, or the like, fused to the DNA sequence coding for the p18AβrP protein, can be expressed. In particular, fusions with green fluorescent protein are simple and sensitive, and thus may be recommended. After selection of cells displaying the expression, it is necessary to examine the above-described properties a) , b), and c) .

The p18AβrP protein can be isolated by extraction of cells expressing the p18AβrP protein by appropriates procedures known to those skilled in the art (using immunoprecipitation with a p18AβrP-specific antibody, for example), followed by purification (using a Sepharose column utilizing the above-mentioned antibody as a carrier, for example).

The present invention further relates to a method of screening cell-death promoting or suppressing substances, which comprises contacting a cell expressing p18AβrP with a test substance in the presence of a differentiation inducing factor, and determining the suppression or enhancement of cell death.

Examples of the method of screening substances which interact with p18AβrP to suppress its function or which suppress cell death by affecting the interaction of p18AβrP with Hsp70 and/or Tid-1 include a method comprising introducing a gene coding the p1BAβrP protein into an appropriate vector, using it to transform a cell, preferably a nerve cell, culturing the transformed cell under the action of a differentiation inducing factor (for example, NGF) and in the presence of a test substance, and in the case of a nerve cell, assaying neurite elongation and cell death , and comparing with the result obtained without the test substance and/or without the differentiation inducing factor (i.e., a control system). Appropriate vectors and cells are known to those skilled in the art and include the above-mentioned examples. In a cell expressing p18AβrP, also in the presence of a differentiation inducing factor at a given concentration, neuritis elongation is suppressed (in the case of a nerve cell) , so that cell death can be observed. On the other hand, the presence of a substance which interacts with p18AβrP to suppress its function or which suppresses cell death by affecting the interaction of p18AβrP with Hsp70 and/or Tid-1 allows growth of neurites (in the case of a nerve cell), avoiding cell death. In such a case, it is likely that the test substance is a substance which interacts with p18AβrP to suppress its function to suppress cell death or which suppresses cell death by affecting the interaction of p18AβrP with Hsp70 and/or Tid-1.

Examples of the method of screening substances which interact with p18AβrP to promote its function or which promote cell death by affecting the interaction of p18AβrP with Hsp70 and/or Tid-1 include a method comprising introducing a gene coding for the p18AβrP protein into an appropriate vector, using it to transform a cell, preferably a nerve cell, culturing the transformed cell is cultured under the action of a differentiation inducing factor and in the presence of a test substance, assaying neurite elongation and cell death, and comparing with the result obtained in the absence of the test substance. Appropriate vectors and cells are known to those skilled in the art and include the above-described examples. When in the presence of a differentiation inducing factor at higher concentrations than employed in screening substances which interact with p18AβrP to suppress its functions, a test substance is added to a nerve cell elongating neurites or a cell causing no cell death, whereby the growth of neurites is suppressed (in the case of nerve cell) or cell death is observed, it is likely that the test substance is a substance which interacts with p18AβrP to enhance its function to promote cell death or which promotes cell death by affecting the interaction of p18AβrP with Hsp70 and/or Tid-1.

Examples of the method of evaluating whether cell death has been caused include a method by a microscopic method as described above.

The present invention further relates to substances which interact with p18AβrP to suppress and promote cell death. Said substances are ones interacting with p18AβrP, and the present invention also includes substances which suppress or promote cell death by affecting the interaction of p18AβrP with Hsp70 and/or Tid-1. Preferably, cell-death suppressing or promoting substances are substances obtained by the above-described screening methods. Such substances may be any kind of molecules, including, for example, proteins, peptides, and small molecules such as other low molecular-weight organic compounds.

The cell-death suppressing substances of the present invention are useful for the prophylaxis and/or treatment of, for example, neurodegenerative diseases in brain associated with cell death of nerve cells, such as Alzheimer' s disease, Down' s syndrome, and other dementias, as well as Huntington's chorea disease, amyotrophic lateral sclerosis, spinocerebellar degenerative disease, Parkinson's disease, and the like. In one embodiment, therefore, the present invention relates to a pharmaceutical composition for the treatment and/or prophylaxis of diseases associated with cell death, containing a cell-death suppressing substance as described above. Usually, pharmaceutical compositions contain pharmaceutically acceptable carriers or excipients. Methods of manufacturing pharmaceutical compositions, dosage forms, and pharmaceutically acceptable carriers or excipients are selected depending upon conditions of subjects, sites to be treated, routes of administration, and others, and can be readily selected by those skilled in the art.

During the above-described screening of the present invention, there was found a 60TRP novel gene coding for the p60TRP protein suppressing cell death and the p60TRP protein coded by the gene.

In a further embodiment, therefore, the present invention relates to a cDNA comprising the base sequence of nucleotides 82 to 1701 of SEQ ID NO:5. The present invention also relates to an mRNA which is complementary to the above-described cDNA. The above-described cDNA is one coding for the protein of SEQ ID NO:6. The amino acid sequence of SEQ ID NO: 6 is of the rat p60TRP protein, and thus the nucleotide sequence (nucleotides 82 to 1701) of SEQ ID NO:5 codes for the rat p60TRP protein. Hereinafter, the rat p60TRP is simply referred to p60TRP. Thus, the p60TRP protein of the present invention has the amino acid sequence of SEQ ID NO:6. The above-described cDNA can be typically obtained by methods employing, for example, RT-PCR procedures, cDNA differential procedures, and others, as described in the section Methods in Examples, which methods are known to those skilled in the art.

In another embodiment, the present invention relates to a DNA coding for a variant p60TRP protein which has one or more amino acids inserted, deleted, or substituted in the amino acid sequence of the p60TRP protein (SEQ ID NO:6) and displays a cell-death suppressing effect similar to that of the protein having the amino acid sequence of SEQ ID NO:6, and to a DNA coding for a variant p60TRP protein which is hybridizable with the above-described DNA under stringent conditions and displays a cell-death suppressing effect similar to that of the protein having the amino acid sequence of SEQ ID NO:6. Stringent conditions are described in many textbooks and the literature and well known to those skilled in the art. Stringent conditions are exemplified by conditions allowing hybridization at 42°C in a solution containing 6X SSC, 5X Denhardt' s reagent, 0.5% SDS, and 100 µg/ml salmon sperm DNA.

The present invention further relates to a DNA having a nucleotide sequence homology of at least more than 60%, preferably 70% or more, more preferably 80% or more, and most preferably 90% or more and less than 100%, based on a cDNA comprising the base sequence of nucleotides 147 to 647 of SEQ ID NO:3.

A protein coded by any one of the DNAs described above is also included in the p60TRP protein of the present invention.

In this specification, a variant p60TRP protein as described above may be designated as a p60TRP protein, as long as it has a cell-death suppressing effect similar to that of the protein having the amino acid sequence of SEQ ID NO:6, that is to say, a p60TRP protein. Additionally, a p60TRP homologous protein from species other than rat, for example, a human p60TRP protein, may be designated as a variant p60TRP or simply p60TRP protein, as long as it has a cell-death suppressing effect similar to that of the protein having the amino acid sequence of SEQ ID NO:6, that is, a p60TRP protein. Such a homologous p60TRP protein has a homology of at least 60% or more, preferably 70% or more, more preferably 80% or more, and most preferably 90% or more and less than 100%, based on the rat p60TRP protein.

In a further embodiment, the present invention relates to a vector containing the DNA coding for the above-described p60TRP protein, including a variant, and also to a transformant such as a cell transformed with the vector.

Vectors which may be used for introducing any one of the above-described DNAs coding for the p60TRP protein to transform a nerve cell utilize a variety of vectors described in the literature and a variety of commercial available vectors, for example, pVgRXR, pIND, pIND/V5-His, pIND/GFP, pcDNA3.1 or pcDNA3.1/myc or pcDNA3.1/His or pcDNA3.1/V5-His or pcDNA3.1-CT-GFP-TOPO®, or virus vectors such as LNCX2. Methods for transformation are known, such as calcium phosphate methods, Super Fector reagent methods, and lipid-mediated methods employing LipofectAMINE reagent or the like. Cells to be used for screening may utilize any kind of cells, preferably nerve cells. Examples of preferred nerve cells include PC12 cell, NB2a, Neuro-2A, B104, SHSY-5Y, primary culture nerve cell, and the like. Construction of the above-described vector, cell transformation, and conditions for culturing cells are determined depending upon individual cells, and are within the knowledge of those skilled in the art or can be readily determined. Such a transformant is also included in the present invention. It is desirable to attach a detectable tag to a p60TRP protein to be expressed, so that the expression of the p60TRP protein can be detected with ease. A tag can be attached at the DNA level: for example, green fluorescent protein DNA, red fluorescent protein DNA, myc gene, or the like, fused to the DNA sequence coding for p60TRP protein, can be expressed. In particular, fusions with green fluorescent protein are simple and sensitive, and thus may be recommended. After selection of cells displaying the expression, it is necessary to examine the cell-death suppressing effect in the screening assay described herein.

The p60TRP protein can be isolated by extraction of cells expressing the p60TRP protein by appropriates procedures known to those skilled in the art (using immunoprecipitation with a p60TRP-specific antibody, for example), followed by purification (using a Sepharose column utilizing the above-mentioned antibody as a carrier, for example).

In a further embodiment, the present invention relates to a pharmaceutical composition for suppressing cell death, containing the above-described p60TRP protein, including a variant, a DNA coding for the protein, a vector containing the DNA coding for the above-described p60TRP protein, including a variant, or a transformant transformed with the vector. In addition, the present invention also relates to a method for suppressing cell death in a subject, characterized by administering one of the materials or the transformant to the subject, and to their use for manufacturing a medicament for suppressing cell death. Methods for manufacturing pharmaceutical compositions, dosage forms, and pharmaceutically acceptable carriers or excipients are selected depending upon conditions of subjects, sites to be treated, routes of administration, and others, and can be readily selected by those skilled in the art. These compositions, methods, and uses for suppressing cell death allow treatment and/or prophylaxis of various neurodegenerative diseases in brain associated with cell death, such as Alzheimer's disease, Down's syndrome, and other dementias, as well as Huntington's chorea disease, amyotrophic lateral sclerosis, spinocerebellar degenerative diseases, Parkinson's disease, and the like. In the above-described treatment, when employing a p60TRP gene or a vector containing the gene, gene therapy is provided, in which it is possible to introduce the gene or vector into cells obtained from a subject, which can be cultured and then return back to the subject, or alternatively the p60TRP gene or vector containing the gene may be introduced directly into a subject.

In addition, the present invention provides substances regulating (promoting or suppressing) the cell-death effect of p60TRP. Specifically, such substances are substances promoting the cell-death suppressing effect of p60TRP by interacting with p60TRP to inhibit cell death signal via p18AβrP (p60TRP agonists) , and substances attenuating the cell-death suppressing effect of p60TRP by interacting with p60TRP to suppress the inhibition of cell death signal via p18AβrP (p60TRP antagonists). These substances regulating the cell-death suppressing effect of p60TRP include, but are limited to, natural or synthetic proteins, peptides, nucleic acids, and the like, and may be natural or synthetic low molecular-weight compounds. Examples of p60TRP agonists are, for example, PP2A, Ran BP5, and other proteins. For screening such agonists and antagonists of p60TRP, two-hybrid systems can be generally employed, as described below.

In addition, for example, a p60TRP agonist can be added to a pharmaceutical composition containing p60TRP to further enhance the cell-death suppressing effect, thereby allowing more effective treatment or prophylaxis of diseases associated with cell death such as Alzheimer's disease.

The cell-death promoting substances of the present invention are useful, for example, for establishing cell death systems and for various researches relating to cell death, e.g. elucidating mechanisms of diseases associated with cell death of nerve cells, such as Alzheimer's disease. Furthermore, the cell-death promoting substances of the present invention are useful for the treatment and/or prophylaxis of diseases resulting from cell hyperproliferation, such as cancers and autoimmune diseases. Therefore, in another embodiment, the present invention relates to a pharmaceutical composition for the treatment and/or prophylaxis of diseases resulting from cell hyperproliferation, containing a cell-death promoting substance as described above. Methods of manufacturing pharmaceutical compositions, dosage forms, and pharmaceutically acceptable carriers or excipients are selected depending upon conditions of subjects, sites to be treated, routes of administration, and others, and can be readily selected by those skilled in the art.

The present invention also relates to a method for the treatment and/or prophylaxis of diseases resulting from cell hyperproliferation in a subject, characterized by administering to the subject a cell-death promoting substance as described above. The present invention also relates to the use of a cell-death promoting substance as described above in manufacturing a medicament for the treatment and/or prophylaxis of diseases resulting from cell hyperproliferation. Gene therapy as described above may be also applied in the treatment and/or prophylaxis of diseases resulting from cell hyperproliferation.

The present invention further relates to a method of examining cell death in cells, particularly nerve cells, and to a method for the diagnosis of diseases associated with such cell death, characterized by examining the level of expression of the p18AβrP gene or p18AβrP protein in cells and tissues obtained from a subject. One may detects the p18AβrP gene in cell and tissue samples obtained from a subject, for example, at the mRNA level, or the p18AβrP protein may be detected in cell samples. Such detection can be achieved by procedures well known to those skilled in the art, such as hybridization employing a probe (preferably, labeled with a radioisotope, fluorophore, enzyme, or the like, for example) complimentary to the p18AβrP gene, or by utilizing the binding to a monoclonal antibody (preferably, labeled with a radioisotope, fluorophore, enzyme, or the like, for example) directed to the p18AβrP protein. In this case, it is possible to determine the intensity of expression of the p18AβrP gene or the amount of the p18AβrP protein by examining the signal intensity of the label.

The present invention also relates to a kit for examining cell death in cells, particularly nerve cells, and to a kit for the diagnosis of diseases associated with such cell death, characterized by examining the level of expression of the p18AβrP gene or p18AβrP protein in cells and tissues obtained from a subject. As components of the kit are included, for example, a probe (preferably, labeled with a radioisotope, fluorophore, enzyme, or the like, for example) complimentary to the p18AβrP gene and/or a monoclonal antibody (preferably, labeled with a radioisotope, fluorophore, enzyme, or the like, for example) directed to the p18AβrP protein. The kit usually has its instructions for operation appended thereto.

### EXAMPLES

The following Examples describe the present invention, but are strictly for the purpose of illustration, and are not intended to be limiting to the present invention.

### I. Experimental Methods

Preparation of rat cells: CG-4 (an oligodendrocyte precursor cell, gifted by professor Kazuhiro IKENAGA, Department of Neuronal Information, National Institute for Physiological Sciences, Okazaki National Research Institutes) was cultured in a medium of DMEM/F-12 (1:1 v/v), supplemented N1 (5 mg/l of insulin, 16.1 mg/l of putrescine, 50 mg/l of transferrin, 4.6 mg/l of D-galactose, 8 mg/l of Na selenite, 2.4 g/l of HCO₃), and 30% (v/v) of B104 cell serum-free medium. For inducing the differentiation to oligodendrocytes, CG-4 cells were cultured for 24 hours and B104 cells without a mitogenic factor, after that 2% FCS (Gibco) was added to enhance survival.

RT-PCR methods: Analysis of mRNA and isolation of p60TRP were conducted using RT-PCR (Heese et al., Eur. J. Neurosci., 15, 79 (2002)). Briefly, total RNA of cells was prepared according to the TRIzol® Reagent protocol (Gibco BRL, NT, USA). After extraction of mRNA with chloroform, the RNA was precipitated by adding an equal volume of isopropyl alcohol to an aqueous layer, rinsed with 75% ethanol, and dissolved in RNase-free water to measure the absorbance (at 260 nm) on a spectrophotometer. Total RNA of each sample (0.2 µg/ml) was first subjected to reverse transcription to cDNA (oligo(dT)-primed-SMART™ cDNA-synthesis (Clontech, Tokyo, Japan) ; Superscript II™ (Gibco BRL, NY, USA) ) , and 0.5 µl aliquot was used for PCR reactions (reaction volume: 25 µl) employing 18AβrP-specific primers (sense: 5'-atgagtgaatggacgaagaaaagccccttagaatgggaggat-3' (SEQ ID NO:1); antisense: 5'-tctgggaagctgaaagatggccttgaataagatcctgaattcggg-3' (SEQ ID NO:2)). The number of cycling reactions employed for amplification of each cDNA was set in the linearity range according to the Elongase™ enzyme mix protocol. Denaturing in the amplification step was carried out at 94°C for one minute, and annealing was carried out, with the specific primers, at 65°C for 50 seconds and then at 68°C for additional one minute longer (65°C, 24 cycles). Rat p60TRP cDNA was prepared by 5' -RACE-RT-PCR using a primer derived from a rat brain cDNA library (pAP3neo, Takara) . Isolation of p60TRP was carried out with the following procedure (Heese et al., Eur. J. Neurosci., 15, 79 (2002)). Briefly, total cellular RNA was isolated according to the TRIzol® Reagent protocol (Gibco BRL). After extraction with chloroform, the RNA was precipitated by adding isopropyl alcohol to an aqueous layer, rinsed with 75% ethanol, and redissolved in RNase-free water and quantified on a spectrophotometer (at 260 nm). Total RNA of each sample (0.2 µg/ml) was first subjected to transcription to cDNA (oligo(dT)-primed-SMART™ cDNA-synthesis; Clontech; Superscript II™ (Gibco)), and 0.5 µl aliquot was used for PCR amplification reactions (reaction volume: 25 µl) employing rat (r) and human (h) p60TRP-specific primers (for isolation: sense: 5'-gcgtaatacgactcactatagggaattcgacgt-3' (SEQ ID NO:9), antisense: 5'-cgcgacgtacgatttaaattaaccctcactaaa-3' (SEQ ID NO:10); r-sense: 5'-atgactggctcaaagaataaggctcgggctcaggctaaactg-3' (SEQ ID NO:11), r-antisense: 5'-ttacattctttcaataatccctttaacttcacggaatatggcagt-3' (SEQ ID NO:12); h-sense: 5'-atggctgggactaagaataagacaagagcccaggccaaaac-3' (SEQ ID NO:13), h-antisense: 5'-cattgtttcaataatctctttaacttccctgaaaatggccatgag-3 (SEQ ID NO:14)). The number of cycles was set in the linearity range according to the Elongase™ enzyme mix protocol (Gibco) . PCR was carried out as follows: denaturing was at 94°C for 0.5 minutes, annealing at 65°C for 50 seconds (annealing temperature), and extending at 68°C for two minutes (annealing temperature: 65°C, 16 cycles).

PCR amplification reactions (60°C, 16 cycles) of a constitutively expressing ribosomal protein S12 were used for measurement of introduced RNA. Controls without reverse transcription employing RNA samples or without RNA were used to ascertain the absence of contamination with DNA. PCR reactions were analyzed by electrophoresing on 1.5% agarose gel, transferring DNA fragments onto a nylon membrane, and allowing to hybridize with a fluorescently labeled DNAprobe. The membrane was subjected to analysis using a FluoroImager 595 (Image Quant ver. 5.0 (Molecular Dynamics, Tokyo, Japan)). In addition to non-parametric statistical analysis (Kruskal-Wallis test), statistical analysis of the results was carried out using analysis of variance (ANOVA) , and errors were expressed by standard errors.

CDNA differential: PCR-Select™ cDNA differential (Clontech) was carried out, in order to determine the difference in expressed mRNA between groups of cells in which Aβ1-42 was added to induce cell death and control cells. Briefly, CG-4 cells were incubated under conditions of fetal calf serum (FCS) ± Aβ1-42 (10 µg/ml) for 60 hours and subjected to cDNA differential (Heese et al., Neurosci. Lett., 288, 37 (2000); Biochem. Biophys. Res. Commun., 289, 924 (2001)). The first-strand synthesis was carried out by converting mRNA of each groups to cDNA by the SMART™-PCR-cDNA synthesis (Clontech) and using a modified oligo-dT primer (a CDS primer) . SMART™-oligonucleotide-anchor and polyA⁺ sequences were used as universal priming sites for cDNA amplification from end to end (LD-PCR). Hybridization of cDNAs derived from the Aβ1-42 treatment cell group and cDNAs derived from the control cell group was performed to remove these cDNAs, and unhybridized cDNAs were referred to as differential cDNAs activated with Aβ1-42. The differential cDNAs were cloned into the TOPO®-T/A cloning vector (Invitrogen) and subjected to identification by southern blotting. The base sequence was analyzed on a sequencer (ABI PRIM™ BigDye™ Terminator Cycle Sequencing Ready Reaction Kit (Perkin-Elemer; sequencer: ABI PRISM Model 310)).

CDNA cloning: After cDNA subtraction, a full-length cDNA from EST sequences of rat p18AβrP was obtained by employing oligonucleotides designed from partial cDNA/EST sequences from a database (http://www.ncbi.nlm.nih.gov) and carrying out the screening using a rat brain cDNA library (ClonCapture Ready™ Super DNA; Clontech, Tokyo, Japan) in 5'-RACE (rapid amplification of cDNA ends) and RT-PCR experiments. A p18AβrP construct for analysis was made by inserting the rat p18AβrP cDNA into the pCR®II-TOPO® T/A cloning vector (Invitrogen, Tokyo, Japan). A p18AβrP Construct for expression (p18AβrP-CT-GFP) was made by inserting pcDNA3.1CT-GFP-TOPO® (Invitrogen, Tokyo, Japan) for the expression of green fluorescent protein (GFP) into the rat p18AβrP cDNA at the C-terminal of p18AβrP.

### Analysis of the p18AβrP cDNA sequences and amino acid:

Analysis of the p18AβrP cDNA and amino acid sequences were carried out using PDB, SwissProt, PIF, and PRF, in addition to the NCBI (National Center for Biotechnology Information) Blastp 2.0 program (Nucleic Acids Res., 25, 3389 (1997)) to un-overlapped GenBank CDS translations and the UniGene database (NCBI) (Nucleic Acids Res., 25, 2289 (1997)). Homology search was carried out using Blast and FASTA (Wisconsin Package ver. 10.0, Genetics Computer Group (GCG), Madison, WI) algorithms, and BestFit (Eisconsin Package Version 10.0, GCG) . Motifs of the amino acid sequence were searched using PROSITE-Profile, and BLOCKS-, ProDom-, PRINTS-, Pfam-and PSORTII-programs (Nucleic Acids Res., 27, 260 (1999), Intellig. Syst. Mol. Biol., 4, 109 (1996); Intellig. Syst. Mol. Biol., 5, 147 (1997)). Phosphorylation sites were searched using NetPhos 2.0 (J. Mol. Biol., 294, 1351 (1999)), and other analyses were carried out using not only the ExPASy www server (http://www.expasy.ch), but also http://www/softberry.com/index.html and the amino acid composition search (AACompIdent) http://kr.expasy.org/tools/aacomp/. The determined p18AβrP cDNA and amino acid sequences are shown in SEQ ID NO:3 (Fig. 1a) and SEQ ID NO:4 (Fig. 1b), respectively. In addition, Fig. 1c shows a comparison of the amino acid sequences of the rat p18AβrP protein of the present invention and human and mouse homologous proteins.

Analysis of p18AβrP expressing tissues: Twenty-two tissues from rats were taken according to usual procedures, to prepare samples: 1 = brain; 2 = heart; 3 = kidney; 4 = spleen; 5 = liver; 6 = colon; 7 = liver; 8 = small intestine; 9 = muscle; 10 = stomach; 11 = testis; 12 = salivary gland; 13 = throid; 14 = adrenal; 15 = pancreas; 16 = ovary; 17 = uterus; 18 = prostate; 19 = skin; 20 = leukocyte; 21 = bone marrow; and 22 = fetus brain (the number corresponds to the lane number in Fig. 4). Tissue samples were prepared by preparing cDNAs form each of the tissue samples in the previously described method (see Section RT-PCR) . In order to examine the tissue-specific expression of p18AβrP, Rapid-Scan™-Gene-Expression panels (Origene Technologies, MD, USA) was used. PCR products were analyzed using a standard 2% DNA electrophoresis agarose E-gel™ (Invitrogen).

Methods of screening cell death suppressing and/or promoting substances - Obtaining of a cell-death suppressing substance p60TRP: In PC12 cells expressing p18AβrP, there were not observed cell differentiation, survival, and neurite elongation, in spite of the addition of NGF, whereas when the expression of a rat cDNA library in these cells resulted in the finding of surviving cells, in spite of the expression of p18AβrP. Thus, by the death trap screening method (Semin. Immunol., 9, 17 (1997)) from these surviving cells, a gene involved in this survival was found with RT-PCR and named a p60TRP gene. Also, the cell-death suppressing protein coded by this gene was named a p60TRP protein.

Analysis of the p60TRP cDNA base sequence and the protein primary structure: Sequence analysis of the p60TRP cDNA and protein was carried out using as a search tool the NCBI (National Center for Biotechnology Information) Blast 2.0 program employing the UniGene database (NCBI) (Nucleic Acids Res., 25, 3389 (1997)) and the GenBank CDS translation + PDB + Swiss Prot + PIR + PRF databases. For homology search, Blast and FASTA (Wisconsin Package Version 10.0, GCG (Genetics Computer Group) algorithms were used, and BestFit (Wisconsin Package Version 10.0 GCG) was used for alignment. The protein sequence was determined employing the ExPASy-www-server (http://www.expasy.ch); softberry: http://www.softberry.com/index.html and the amino acid composition search (AACompIdent): http://kr.expasy.org/tools/aacomp/. Motifs of the amino acid sequence were searched using PROSITE Profile, BLOCKS-ProDom-PRINTS-Pfam-and PSORT II-programs (Nucleic Acids Res., 27, 260 (1999); Mol. Biol., 4, 109 (1996); Mol. Biol., 5, 147 (1997)). Phosphorylation sites were searched using the NetPhos 2. 0 protein phosphorylation search server (J. Mol. Biol., 294, 1351 (1991)).

Analysis of p60TRP expression in tissues: Analysis of the tissue-specific gene expression of p60TRP utilized Rapid-Scan™-Gene-Expression panels (Origene Technologies, Rockville, MD, USA), and tissue cDNAs were subjected to semi-quantitative RT-PCR analysis. PCR products were analyzed using a standard 1.5% DNA electrophoresis agarose E gel™ (Invitrogen, Brain Aging, 2, 30 (2002)).

Cell culture: B104, rat neuroblastoma cell (available from Professor Kazuhiro Ikenaka, National Institute for Physiological Sciences, Okazaki National Research Institutes, for example) , and PC12 cell (available fromATCC (American Type Culture Collection) , ATCC No. CRL-1721, for example) were cultured in Dulbecco's Modified Eagle Medium (D-MEM)/F-12 (1:1) containing N2 supplemented with 10% fetal calf serum (FCS; Gibco BRL, Grand Island, NY, USA), and CHO (Chinese hamster ovary) cell line was cultured in DMEM supplemented with 10% FCS, under the condition of 5% CO₂/95% air at 37°C. CG-4 (an oligodendrocyte precursor cell) was cultured in a medium of DMEM/F-12 (1:1 v/v), supplemented N1 (5 mg/l of insulin, 16.1 mg/l of putrescine, 50 mg/l of transferrin, 4.6 mg/l of D-galactose, 8 mg/l of Na selenite, 2.4 g/l of HCO₃), and 30% (v/v) of B104 cell serum-free medium. For inducing the differentiation to oligodendrocytes, CG-4 cells were cultured for 24 hours and B104 cells without a mitogenic factor, after that 2% FCS (Gibco) was added to enhance survival. For inducing cell death, cells were cultured for 60 to 72 hours with FCS ± Aβ (Peptide Institute, Inc., Osaka, Japan; dissolved in a serum-free solution, in 1 mg/ml phosphate buffered saline (PBS), pH 7.4, to 10 µg/ml, followed by incubation at 37°C for 24 hours), and then surviving cells were measured using a Promega kit (CellTiter96® AQᵤₑₒᵤₛ One Solution cell proliferation assay) . For inducing neurite elongation, PC 12 cells were subjected to gene transfer with p18AβrP-CT-GFP or a control vector, and then induced with NGF (50 ng/ml) for 24 hours.

Gene transfer into cells: Constructed were p18AβrP-CT-GFP having GFP (green fluorescent protein) introduced at the C-terminal of p18AβrP; p60TRP-CT-GFP and p60TRP-CT-DsRed1 having, respectively, GFP (pcDNA3.1CT-GFP-TOPO® (Invitrogen) or DsRed1 (NheI and HindIII restriction enzyme cloning sites, Clontech, Tokyo, Japan) introduced at the C-terminal of the rat p60TRP cDNA. Additionally, p60TRP was subcloned into pIRES2-EGFP (Clontech), in order to allow co-expression with GFP from the same mRNA (p60TRP-IRES-GFP). P18AβrP-CT-GFP or p60TRP-DsRed1, p60TRP-GFP, p60TRP-IRES-GFP, and GFP (Clontech, Tokyo, Japan) expression vector, or an empty vector (control) was transiently gene transferred into CHO cell (available from ATCC, ATCC No. CCL-61, for example) and PC12 cell (available from ATCC, ATCC No. CCL-1271, for example) (SuperFector, B-Bridge, San Jose, CA, USA), which were cultured using a D-MEM/F12 (1: 1) /N2 containing culture medium supplemented with 10% fetal calf serum (FCS, Gibco BRL, Tokyo) at 37°C under the condition of 5% CO₂/95% air. The efficiency of gene transfer was verified by co-expression of GFP and p53-/PKC-DsRed1 (Clontech) , so as to be always 50 to 60% (Eur. J. Neurosci., 15, 79 (2002)). Cell death/survival was evaluated under a fluorescent microscope (Olympus IX70, Olympus, Tokyo, Japan) at 48 hours after gene transfer. Further, 24 hours later, NGF (murine NGF 2.5S, 50 ng/ml; Invitrogen) was added to the PC12 cells, and after 120 hours, surviving cells were measured in the CellTiter96® AQᵤₑₒᵤₛ Assay (Promega, Madison, WI, USA) and observed under a fluorescent microscope.

Two-Hybrid system: A yeast strain MaV203 (available from Invitrogen, for example) was used. Rat p18AβrP or rat p60TRP was subcloned into the pDEST™32 vector (Invitrogen) having a GAL4 DNA binding domain from pENTR/D-TOPO®. Also, pEXP-AD502 was used as an expression vector for an activation domain having a ProQuest™ two-hybrid rat brain cDNA library (Invitrogen). For selection for activity, three reporter genes, HIS3, URA3, and lacZ, were used. Each of these genes was stably integrated into a yeast gene at a different site, and the promoter region of the HIS3, lacZ, and URA3 is different except for the GAL4 binding domain. It is reported that the ProQuest™ Two-Hybrid System enables three independent transcriptions to take place from respective separate chromosomes, thereby giving reduced false-positive reactions, as compared to standard two-hybrid systems. The induction of the HIS3 and URA3 reporter genes is caused depending upon the two-hybrid, and respectively enables cells to grow also on a plate lacking histidine or uracil, so that cells can be discriminated. On the other hand, the induction of the lacZ gene can be done with X-gal (5-brome-4-chloro-3-indolyl-β-D-galactopyranoside), resulting in blue color. In addition, due to the toxicity resulting from the conversion of 5-fluoroorotate (5-FOA) to 5-fluorouracil, the induction of URA3 enables cells to grow in a culture medium lacking uracil and inhibits the growth in a culture medium containing 5-FOA. Therefore, this system enables the screening of four phenotypes, that is, proteins displaying the true interaction by means of His (3AT®), β-gal, Ura⁺ and 5-FOA^{s} and thus the elimination of false-positive reactions. The use of the ARS/CEN vector also can reduce the expression level and the toxicity. Positive clones can be identified by retransformation. When an interacting protein is contained, a yeast cell binds to db-rat p60TRP or db-rat p18AβrP and ad-Y (wherein Y is, for example, PP2A or TID-1). The plasmid DNA from a yeast strain containing the above can be introduced into an E. coli cell by electroporation, and the transformant can be selected with ampicillin or the db-rat p18AβrP can be selected with gentamycin. The plasmid DNA of these E. coli cells, ad-Y (wherein Y is, for example, PP2A or TID-1), can be introduce into MaV203 together with pDBleu or db-rat p18Aβr or db-rat p60TRP, and the induction of the reporter genes by db-rat p18Aβr or pdb-rat p60TRP will give true positive reactions.

Production of anti-p60TRP antibody: A rabbit anti-p60TRP antibody was prepared and used which has a high affinity for the N-terminal domain of p60TRP (amino acid (aa)-35 to aa-45: RGAGKNRDKGK-cys).

Protein immunoprecipitation and western blot analysis: 24 hours after transient gene transfer of p60TRP-IRES-GFP into about 5 x 10⁶ PC12 cells, culturing was continued for additional 48 hours. After that, the cells were washed twice with Tris-saline buffer (TBS, pH 7.2) and lysed at 4°C with 0.5 ml of an ice-cooled buffer (150 mM NaCl, 50mM Tris-HCl pH 8.0, 1% NP40, 2% glycerol, 1mM PMSF, 10 µg/ml aprotinin, 1 µg/ml leupeptin, 0.5mM Na vanadate), and the nucleus was removed by centrifugation at 4°C. After incubating with the antibody (anti-p60TRP) at 4°C for 2 hours, 500 µl of 50% Protein-A Sepharose® CL-4B was added and incubation was carried out for additional 2 hours. Immunoprecipitate was washed three times, and 50 µl of the Laemmli-protein buffer (Bio-Rad, Tokyo, Japan) was added, followed by western blot analysis. Briefly, proteins were electrophoresed on 10% polyacrylamide gel to separate. The proteins were transferred onto a polyvinylidene fluoride membrane (PVDF) (Bio-Rad, Tokyo, Japan), and then immunoreacted with an anti-PP2A (regulatory subunit) antibody (SantaCruz, CA, USA), and a secondary fluorescein-conjugated anti-goat antibody and a tertiary alkaline phosphatase-conjugated anti-fluorescein antibody were added and incubated with the substrate of alkaline phosphatase (the ECF™ western blotting kit, Amersham/Pharmacia, Tokyo, Japan).

Evaluation of cell death: PC12 cells were cultured to a confluency of 50 to 80% and treated with trypsin 24 hours prior to gene transfer, diluted 5 times in a fresh medium lacking antibiotics (1 to 3 x 10⁵ cells/ml), and transferred into a 24-well plate (500 µl/well) and cultured. In experiments for evaluating cell death, investigation was made on cells expressing no GFP (control-1), cells expressing only GFP (control-2), and cells having the p60TRP gene knock-outed by SiRNA. The gene knock-out by SiRNA, was carried out employing Oligofectamine and introducing 0.5 µg of siRNA per well (Brain Aging, 2, 44 (2002)). The efficiency of gene transfer was determined under a fluorescent microscope after the co-expression of 1 µg of a GFP expression vector and 0.2 µg of siRNA (Mol. Brain Res., 104, 127 (2002) ; Nature, 411, 494 (2001)) . The percentage of cell death/survival by SiRNA was determined 48 hours after expression, by the Cell-Titer 96® AQᵤₑₒᵤₛ One solution assay (Promega, Madison, WI: Neurosci. Lett., 288, 37 (2000)). The siRNA sequence for p60TRP utilized the sequence of nt 310 to nt 330 relative to the start codon. P60TRP-specific 21-nucleic acid duplex siRNA was obtained from Dharmacon Research (Lafayetta, CO, USA; B-Bridge International, Tokyo, Japan).

The experimental procedures described above are typical as known in the art, and those skilled in the art will recognize or should easily conceive their variations and alternative procedures.

### II. Experimental Results

The experimental results according to the present invention will be described as follows:
(1) The base sequence of the rat p18AβrP cDNA of the present invention is homologous to that of human and mouse sequences, and their amino acid sequences are compared and shown in Fig. 1c. Accordingly, it has turned out that the base sequence of the rat p18AβrP cDNA of the present invention is a novel sequence that is different from the human base sequence described in the above-mentioned literature and sequences found in the Gene Data Bank, and that as can be seen from Fig. 1c, the amino acid sequence of the p18AβrP protein is a novel sequence that is different from that of human and mouse sequences.
(2) It was shown that the expression of p18AβrP in rat CG4 oligodendrocyte lysates was increased by Aβ1-42, when examined by Southern blotting after addition of Aβ1-42 (10 µg/ml) at 37°C for 60 hours (see, Fig. 2 left panel): lane 1, control; lane 2, Aβ treatment (Aβ). The amount of transcription of the p18AβrP mRNA, densitometric quantification resulting in finding to increase by the addition of Aβ1-42 (see, Fig. 2 right panel).
(3) In PC12 cells subjected to differentiation induced by NGF, p18AβrP gave rise to suppressed elongation of neurites and cell death. For the purpose of investigating functions of p18AβrP in nerve cells, a gene of a protein having GFP fused to the C-terminal of p18AβrP was introduced into PC 12 cells, and its expression was identified after 48 hours. Subsequently, 24 hours later, NGF (50 ng/ml) was added and fluorescent microscopic observations were made after 120 hours, with the result that as shown in Fig. 3, elongation of neurites was suppressed and cell death was observed in spite of the presence of NGF, and 2 weeks later, all the cells were killed. On the contrary, in cells having no expression of the gene (having no fluorescence), NGF clearly promoted neurite elongation and allowed survival of the cells. Some of these patterns are shown in Fig. 3, Panels a to i. For example, it was observed in Panels c and f that p18AβrP-positive cells emitting GFP fluorescence as indicated by the arrow were shrinked, caused cell death, and floating. It was also observed that the cell indicated by the arrow in Panel d retained the cellular morphology, while the neurite elongation was suppressed.
(4) The Two-Hybrid system as explained above was employed to screen a protein or proteins interacting with p18AβrP of the present invention, with the result that such a protein was found to interact with the heat shock protein hsp70 and the tumor suppressor protein Tid-1. It is likely that effects of inducing cell death and suppressing cell differentiation as described above resulted from such interaction.
(5) Non-quantitative analysis of mRNA expression was carried out by RT-PCR in terms of the expression of p18AβrP mRNA in rat various tissues. As shown in Fig. 4, its expression was identified in twenty-two organs and tissues, including brain.
(6) Isolation, Characterization of p60TRP, and Expression in Tissues and Cells
   After gene transfer of p18AβrP and a rat brain cDNA library, p60TRP was found in surviving cells by RT-PCR (Fig. 7) . The base sequence of a rat cDNA of this novel gene is shown in SEQ ID NO:5 and Fig. 5A, and the amino acid sequence in SEQ ID NO:6 and Fig. 5B, while for comparison, its homologous human gene cDNA is shown in SEQ ID NO:7 and Fig. 6A, and the amino acid sequence in SEQ ID NO:8 and Fig. 6B. The protein coded by the rat cDNA (nucleotides 82 to 1701 of SEQ ID NO:5) is a protein consisting of 539 amino acids of SEQ ID NO:6 and having a molecular weight of about 59.72 kDa. The base sequence of this rat p60TRP cDNA and its amino acid sequence are clearly different from that of human sequences, and thus are novel sequences. The amino acid homology between the rat p60TRP and human homologous p60TRP proteins suggests that the human homologous p60TRP protein also possesses a cell-death suppressing effect similar to that of the rat p60TRP protein. From their sequences, p60TRP proteins likely constitutes a novel protein family and have a bHLH domain (amino acids 491 to 507 of the sequence of SEQ ID NO:6). Members of the p60TRP protein family having such a domain include O43168, Q96D09, QBVZ3, Q9CVV3, Q9H969, Q920R4, Q9BE11, Q9C0G2, Q9CXQ7, Q9UJC4, Q8R095, O60267, Q9NPE4/Q9UH62, Q9NTS2, Q9BTM6, Q9H2Q0, Q9P291, Q9NWJ13, Q9CX19, Q9DC32, Q9CZ87, Q9CUN3, Q9D0L7, Q9CS81, and Q9CX83.
   Further, RT-PCR was employed to search the expression pattern in tissues, with the result that the mRNA was identified in brain, kidney, and spleen at high expression levels, and also in heart and skeletal muscle, whereas no expression was detected in lung and liver (Fig. 8) . Fig. 9 shows fluorescence images of p60TRP-GFP and p60TRP-DsRed1 in CHO cells. In the CHO cells, the p60TRP-GFP or p60TRP-DsRed1 fusion protein was localized particularly in the cytoplasm (Fig. 9 Panels A and B) and also existed in the nucleus (Fig. 9 Panels C to F).
   In addition, p60TRP did not affect the neurite elongation induced by NGF in PC12 cells. As shown in Fig. 10, when NGF (50 ng/ml) was added 24 hours after p60TRP was expressed in PC12 cells, which were cultured for additional 120 hours, the fact that the neurite-elongating effect of NGF was not suppressed was able to be observed in cells co-expressing p60TRP and GFP.
(7) Proteins Interacting with P60TRP The Two-Hybrid system revealed the interaction of two proteins with p60TRP, i.e., PP2A (protein-phosphatase 2A) responsible for a crucial dephosphorylation reaction in the intracellular signal transduction and RanBP5 (Ran-binding protein 5) involved in the transport of the bHLH transcription factor from the cytoplasm into the nucleus. As shown in Fig. 11, immunoprecipitation and its western blot analysis showed that p60TRP binds to PP2A. It is likely that p60TRP interacts with these proteins and inhibits the cell-death signal via p18AβrP, thereby leading to inhibiting cell death.
(8) Immunoprecipitation of P60TRP and PP2A Fig. 11 represents the result of western blot analysis of immunoprecipitates of p60TRP and PP2A. It was ascertained by immunoprecipitation experiments that p60TRP expressed in PC12 cells was co-precipitated at about 60 kDa by interacting and complexing with PP2A.
(9) Decrease in Percent Cell Survival by P60TRP Gene Knock-out

Gene knock-out by p60TRP-specific siRNA significantly reduced the percentage of surviving PC12 cells, as compared to control groups. That is, as shown in Fig. 12, the control-1 displayed a percent survival of 100% and the control-2 GFP did not change the percent survival with the percentage being 102%, whereas p60TRP-siRNA with knock-outed p60TRP significantly reduced the percentage of surviving cells to 76% (p < 0.05) . This further ascertained the cell-death suppressing effect of the p60TRP protein.

The present invention thus has been described in particular and in detail with reference to the Examples. However, it is possible for those skilled in the art to make modifications and variations other than described above, such as, for example, easy selection of appropriate cell lines, to carry out the invention.

### INDUSTRIAL APPLICABILITY

According to the present invention, a novel gene p18AβrP was found whose expression was increased in oligodendrocytes by amyloid-β protein (hereinafter refereed to Aβ) and its functions were demonstrated. That is, it has turned out that the present gene and its product, p18AβrP protein, possess novel functions of suppressing the promotion of neurite elongation by neurotrophic factors and the sustaining of cell death to promote cell death by interacting with the heat shock protein Hsp70 and the tumor suppressor protein Tid-1. Therefore, the present invention provides screening systems in which these are applied, substances involved in promoting and suppressing cell death which are obtainable using such screening systems, diagnosis and prophylaxis of diseases employing them. By the above-described screening system, the present invention further has found, for the first time, a rat cell-death suppressing protein p60TRP and its coding gene, and also identified operations and effects of the p60TRP protein, i.e. an effect of suppressing cell death, for the first time. Therefore, the present invention provides diagnosis and prophylaxis of diseases associated with cell death employing them.

### SEOUENCE LISTING FREE TEXT

### SEQ ID NO: 1

A sense primer to amplify p18AβrP cDNA.

### SEQ ID NO: 2

An antisense primer to amplify p18AβrP cDNA.

### SEQ ID NO: 3

A nucleotide sequence of cDNA encoding p18AβrP protein.

### SEQ ID NO: 4

An amino acid sequence of p18AβrP protein.

### SEQ ID NO: 5

A nucleotide sequence of cDNA encoding rat p60TRP protein.

### SEQ ID NO: 6

An amino acid sequence of rat p60TRP protein.

### SEQ ID NO: 7

A nucleotide sequence of cDNA encoding human p60TRP protein.

### SEQ ID NO: 8

An amino acid sequence of human p60TRP protein.

### SEQ ID NO: 9

A sense primer (specific to rat/human p60TRP) to amplify p60TRP cDNA.

### SEQ ID NO: 10

An antisense primer (specific to rat/human p60TRP) to amplify p60TRP cDNA.

### SEQ ID NO: 11

A sense primer (specific to rat p60TRP) to amplify p60TRP cDNA.

### SEQ ID NO: 12

An antisense primer (specific to rat p60TRP) to amplify p60TRP cDNA.

### SEQ ID NO: 13

A sense primer (specific to human p60TRP) to amplify p60TRP cDNA.

### SEQ ID NO: 14

An antisense primer (specific to human p60TRP) to amplify p60TRP cDNA.

## Claims

1. A p18AβrP cDNA comprising the base sequence of nucleotides 147 to 647 of SEQ ID NO:3.

2. An mRNA which is complementary to the cDNA according to claim 1.

3. A p18AβrP protein having the amino acid sequence of SEQ ID NO:4.

4. A DNA coding for a p18AβrP protein which has one or more amino acids inserted, deleted, or substituted in the protein according to claim 3 and possesses properties of:
a) increasing the expression by Aβ,
b) interacting with Hsp70 and/or Tid-1, and
c) inhibiting the cell differentiation.

5. A DNA which is hybridizable under stringent conditions with the DNA according to claim 1 and codes for a p18AβrP protein possessing properties of:
a) increasing the expression by Aβ,
b) interacting with Hsp70 and/or Tid-1, and
c) inhibiting the cell differentiation.

6. A p18AβrP protein coded by the DNA according to claim 4 or 5.

7. A vector containing the DNA according to any one of claim 1, 4 and 5.

8. A transformant which has undergone gene transfer by means of the vector according to claim 7.

9. A method of screening cell-death promoting or suppressing substances, which comprises contacting a cell expressing p18AβrP with a test substance in the presence of a differentiation inducing factor, and determining the suppression or promotion of cell death.

10. A substance interacting with p18AβrP to suppress cell death.

11. A substance interacting with p18AβrP to suppress cell death, which is found by the cell assay system according to claim 9.

12. A substance according to claim 10 or 11, which suppresses cell death by affecting the interaction of p18AβrP with Hsp70 and/or Tid-1.

13. A cDNA comprising the base sequence of nucleotides 82 to 1701 of SEQ ID NO:5.

14. An mRNA which is complementary to the cDNA according to claim 13.

15. A protein having the amino acid sequence of SEQ ID NO:6.

16. A DNA coding for a protein which has one or more amino acids inserted, deleted, or substituted in the protein according to claim 15 and possesses a cell-death suppressing effect similar to that of the protein according to claim 15.

17. A DNA which is hybridizable under stringent conditions with the DNA according to claim 13 and codes for a protein possessing a cell-death suppressing effect similar to that of the protein according to claim 15.

18. A protein coded by the DNA according to claim 16 or 17 and possessing a cell-death suppressing effect similar to that of the protein according to claim 15.

19. A protein according to claim 15, which is p60TRP, or its variant protein possessing a cell-death suppressing effect similar to that of p60TRP.

20. A vector containing the DNA according to any one of claim 13, 16 and 17.

21. A transformant which has undergone gene transfer by means of the vector according to claim 20.

22. A pharmaceutical composition for the treatment of diseases associated with cell death, which contains a protein, DNA, vector, or transformant according to any one of claims 10 to 21.

23. A substance interacting with p18AβrP to promote cell death.

24. A substance interacting with p18AβrP to promote cell death, which is found by the cell assay system according to claim 9.

25. A substance according to claim 23 or 24, which promotes cell death by affecting the interaction of p18AβrP with Hsp70 and/or Tid-1.

26. A pharmaceutical composition for the treatment and/or prophylaxis of diseases resulting from cell hyperproliferation, which contains a substance according to any one of claims 23 to 25.

27. A method for the diagnosis of diseases associated with cell death, which comprises determining the level of expression of the p18AβrP gene or p18AβrP protein in cells or tissues obtained from a subject.

28. A kit for the diagnosis of diseases associated with cell death, **characterized by** determining the level of expression of the p18AβrP gene or p18AβrP protein in cells or tissues obtained from a subject.

29. A substance which enhances the cell-death suppressing effect of p60TRP by interacting with p60TRP to inhibit the cell-death signal via p18AβrP.

30. A substance which attenuates the cell-death suppressing effect of p60TRP by interacting with p60TRP to inhibit the suppression of the cell-death signal via p18AβrP.

31. A pharmaceutical composition according to claim 22, which further contains a substance of claim 29 enhancing the cell-death suppressing effect of p60TRP.
